(19)

**Europäisches Patentamt**

**European Patent Office**

**Office européen des brevets**

(11) **EP 4 721 713 A1**

(12) **EUROPEAN PATENT APPLICATION**

(43) Date of publication:
08.04.2026 Bulletin 2026/15

(21) Application number: 24203929.5

(22) Date of filing: 01.10.2024

(51) International Patent Classification (IPC):
*A61F 13/15* (2006.01)   *A61F 13/537* (2006.01)
*A61F 13/84* (2006.01)   *A61L 15/26* (2006.01)
*A61L 15/46* (2006.01)

(52) Cooperative Patent Classification (CPC):
(C-Sets available)
**A61F 13/537; A61F 13/15617; A61F 13/53747;
A61F 13/5376; A61F 13/8405; A61L 15/20;
A61L 15/28; A61L 15/46;** A61L 2300/21;
A61L 2300/404   (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **The Procter & Gamble Company
Cincinnati, OH 45202 (US)**

(72) Inventors:
• **Kamphus, Juliane**
  **65824 Schwalbach am Taunus (DE)**
• **Couturier, Jean-Phillipe**
  **65824 Schwalbach am Taunus (DE)**
• **Benner, Andrea Lieselotte**
  **65824 Schwalbach am Taunus (DE)**
• **Stelzig, Lutz**
  **65824 Schwalbach am Taunus (DE)**

• **Hollenberg, Doris**
  **65824 Schwalbach am Taunus (DE)**
• **Schwamb, Laura**
  **65824 Schwalbach am Taunus (DE)**
• **Simonyan, Arsen Arsenov**
  **65824 Schwalbach am Taunus (DE)**
• **Borgmann, Carolin Michaela**
  **65824 Schwalbach am Taunus (DE)**
• **Chatterjee, Aniruddha**
  **65824 Schwalbach am Taunus (DE)**
• **Fillbach, Tim**
  **65824 Schwalbach am Taunus (DE)**
• **Richter, Meike**
  **53881 Euskirchen (DE)**

(74) Representative: **P&G Patent Germany
Procter & Gamble Service GmbH
Sulzbacher Straße 40
65824 Schwalbach am Taunus (DE)**

(54) **ABSORBENT ARTICLE AND METHOD FOR MAKING AN ABSORBENT ARTICLE**

(57)   The invention relates an absorbent article and a process for making an absorbent article. The absorbent article comprises an acquisition and distribution system comprising at least one acquisition and/ or distribution layer exhibiting an Acid Equivalent value of from 0.050 mmol/g to 6.000 mmol/g.

Fig. 1

**(Cont. next page)**

(52) Cooperative Patent Classification (CPC): (Cont.)

C-Sets
**A61L 15/28, C08L 1/00**

**Description**

FIELD OF THE INVENTION

The present invention relates to an absorbent article comprising an acquisition and distribution system with one or more monomeric acids and a method for making such an absorbent article.

BACKGROUND OF THE INVENTION

Absorbent articles, such as diapers and pants, are well known. While their use is widely accepted, some wearers or caretakers have concerns regarding skin health when using such articles. Skin irritation associated with absorbent articles is often referred to as "diaper rash". Especially for delicate skin of babies, skin health is important. However, skin health is generally a focus for many consumers, also when the absorbent articles are used on toddlers and adults.
Skin irritation can inter alia be caused by high levels of humidity inside the absorbent article during use, and by urine and feces deposited inside the absorbent article. The likelihood of skin irritation thereby increases with prolonged wearing time of the absorbent article.
A known means to address and improve skin health, is the application of lotion on the body-facing surface of the topsheet of the absorbent article. Such lotions often comprise skin-care components. During use of the article, at least a part of the lotion is intended to transfer to the wearer's skin, to provide a certain protection against skin irritation.
However, due to its typically hydrophobic nature, the application of lotion on the topsheet of an absorbent article may negatively impact the acquisition of liquid (such as urine) through the topsheet. Also, a part of the lotion may migrate through the topsheet into the absorbent article during use - or even during storage of the absorbent article prior to use, especially at higher temperatures. Once inside the absorbent article, the lotion can further adversely impact proper acquisition and storage of urine, e.g., within the absorbent material of the absorbent core. Consequently, the use of lotion or other (hydrophobic) substances on the body-facing surface of the topsheet may not only have positive effects on skin care but may also result in some effects which negatively impact skin health, e.g., by prolonging the time which the urine stays on the topsheet before being absorbed through the topsheet, or by slower liquid acquisition and storage inside the absorbent article.
Thus, there remains a need for absorbent articles which can help to reduce the likelihood of diaper rashes.

SUMMARY OF THE INVENTION

The present invention relates to an absorbent article comprising a liquid permeable topsheet, a liquid impermeable backsheet, an absorbent core, and an acquisition and distribution system comprising an acquisition and/ or distribution layer. The absorbent core is provided between the topsheet and the backsheet. The acquisition and distribution system is provided between the topsheet and the absorbent core. The acquisition and/ or distribution layer exhibits an Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein.
The present invention further relates to a process for making an absorbent article comprising a liquid permeable topsheet, a liquid impermeable backsheet, an absorbent core, and an acquisition and distribution system comprising an acquisition and/ or distribution layer. The absorbent core is provided between the topsheet and the backsheet. The acquisition and distribution system is provided between the topsheet and the absorbent core. The process comprises the step of

a) applying one or more monomeric acids to the acquisition and/or distribution layer in an amount such that the acquisition and/or distribution layer exhibits an Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein.

The one or more monomeric acids are applied to the acquisition and/or distribution layer prior to the acquisition and distribution system (38) being provided between the topsheet (26) and the absorbent core (30).
The absorbent articles provided help to improve the wearer's skin condition and thus to reduce the likelihood of diaper rashes.

BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:

Fig. 1 is a plan view of an example absorbent article in the form of a taped diaper, garment-facing surface facing the viewer, in a flat laid-out state;

Fig. 2 is a plan view of the example absorbent article of Fig. 1, body-facing surface facing the viewer, in a flat laid-out state;

Fig. 3 is a front perspective view of the absorbent article of Figs. 1 and 2 in a fastened position;

Fig. 4 is a front perspective view of an absorbent article in the form of a pant;

Fig. 5 is a rear perspective view of the absorbent article of Fig. 4;

Fig. 6 is a plan view of the absorbent article of Fig. 4, laid flat, with a garment-facing surface facing the viewer;

Fig. 7 is a cross-sectional view of the absorbent article taken about line 7-7 of Fig. 6;

Fig. 8 is a cross-sectional view of the absorbent article taken about line 8-8 of Fig. 6;

Fig. 9 is a plan view of an example absorbent core or an absorbent article;

Fig. 10 is a cross-sectional view, taken about line 10-10, of the absorbent core of Fig. 9;

Fig. 11 is a cross-sectional view, taken about line 11-11, of the absorbent core of Fig. 10;

Fig. 12 is a plan view of an example absorbent article of the present disclosure that is a sanitary napkin;

Fig. 13 is an example cross-sectional view taken within a front waist region of an absorbent article;

Fig. 14 is an example cross-sectional view taken within a crotch region of an absorbent article;

Fig. 15 is an example cross-sectional view taken within a back waist region of an absorbent article;

FIG. 16 illustrates an apparatus used in the Modified Fluid Acquisition Test;

FIG. 17A is a side view of the curved component used in the Modified Fluid Acquisition Test;

FIG. 17B is an end view of the curved component of FIG. 17A;

FIG. 17C is a bottom view of the curved component of FIG. 17A;

FIG. 17D is a bottom perspective view of the curved component of FIG. 17A;

FIG. 17E is a top perspective view of the curved component of FIG. 17A;

FIG. 18A illustrates a top plate assembly used in the Modified Fluid Acquisition Test;

FIG. 18B illustrates equipment used in the Modified Fluid Acquisition Test;

Figure 19 shows an equipment assembly used in the Post Acquisition Collagen Rewet Test Method;

Figure 20 shows an equipment assembly used in the Fixed Height Frit Absorption (FHFA) Test Methods;

Figures 21 and 22 show partially schematic views of an equipment assembly used in the In-Plane Radial Permeability (IPRP) Test described herein.

DETAILED DESCRIPTION OF THE INVENTION

Definitions

"Absorbent article" refers to devices that absorb and contain body exudates, particularly urine and other water-containing liquids, and, more specifically, refers to devices that are placed against or in proximity to the body of the wearer to absorb and contain the various exudates discharged from the body. Absorbent articles may include diapers (diapers for babies and infants and diapers to address adult incontinence), pants (pants for babies and infants and pants to address adult incontinence), disposable absorbent inserts for diapers and pants having a re-usable outer cover), feminine care absorbent articles such as sanitary napkins or pantiliners. As used herein, the term "exudates" includes, but is not limited to, urine, blood, vaginal discharges, sweat and fecal matter. Preferred absorbent articles of the present invention are diapers, pants and inserts. Also, preferred absorbent articles of the present invention are disposable absorbent articles, more preferably disposable diapers, disposable pants and disposable absorbent inserts.

"Absorbent core" is used herein to refer to a structure intended to be disposed between a topsheet and backsheet of an absorbent article for absorbing and storing liquid received by the absorbent article.

"Airfelt" is used herein to refer to comminuted wood pulp, which is a form of cellulosic fiber.

"Disposable" is used in its ordinary sense to mean an article that is disposed or discarded after a limited number of usage events over varying lengths of time, for example, less than 10 events, less than 5 events, or less than 2 events. If the disposable absorbent article is a diaper, a pant, absorbent insert, sanitary napkin, sanitary pad or wet wipe for personal hygiene use, the disposable absorbent article is most often intended to be disposed after single use. The used and disposed absorbent article may or may not be subsequently recycled.

"Diaper" and "pant" refers to an absorbent article generally worn by babies, infants and incontinent persons about the lower torso so as to encircle the waist and legs of the wearer and that is specifically adapted to receive and contain urinary and fecal waste. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be pre-formed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

"Superabsorbent polymer material" ("SAP material") is used herein to refer to crosslinked polymeric materials that can absorb at least 7 times their weight of an aqueous 0.9 weight-% saline solution as measured using the Centrifuge Retention Capacity tests set out below. Superabsorbent polymer material of the present invention contains polymers that comprise as monomer groups acrylic acid and/or acrylate and/or methacrylic acid and/or methacrylate. The SAP material is preferably provided in the form of superabsorbent polymer particles (SAP particles) and/or in the form of superabsorbent fibers (SAF).

"Superabsorbent polymer particles" ("SAP particles") is used herein to refer to superabsorbent polymer material that is in particulate form so as to be flowable in the dry state. Superabsorbent polymer particles are distinguished from the superabsorbent fibers of the present invention in that their ratio of largest to smallest dimension is not more than 10 to 1. Superabsorbent polymer particles may for example be in the form of granules, spheres, flakes or agglomerates. The SAP particles may have a CRC value of at least 10 g/g, or of at least 20 g/g, or more preferably of at least 28 g/g according to the CRC test method set out below. The SAP particles may have a CRC value of about 32 g/g or less, or more preferably, in the range between 28 g/g and 32 g/g.

"Superabsorbent polymer fibers" ("SAF") is used herein to refer to superabsorbent polymer material that is in fiber form in the dry state. Superabsorbent fibers are distinguished from the superabsorbent polymer particles of the present invention in that their ratio of largest to smallest dimension is more than 10 to 1, or more preferably more than 100 to 1. The SAP may have a SAF-CRC value of at least 7 g/g, or of at least 12 g/g, or more preferably of at least 20 g/g according to the SAF-CRC test method set out below. The SAP may have a SAF-CRC value of about 32 g/g or less, or more preferably, in the range between 15 g/g and 30 g/g.

As used herein, the term "nonwoven web" refers to a material which is a manufactured web/layer of directionally or randomly oriented fibers or filaments. The fibers or filaments may be of natural or man-made origin. Natural fibers may be selected from the group consisting of wheat straw fibers, rice straw fibers, flax fibers, bamboo fibers, wood pulp fibers, cotton fibers, jute fibers, hemp fibers, sisal fibers, bagasse fibers, Hesper aloe fibers, miscanthus, marine or fresh water algae/seaweeds, silk fibers, wool fibers, and combinations thereof. Another group of fibers may also be regenerated cellulose fibers, such as viscose, Lyocell (Tencel®), rayon, modal, cellulose acetate fibers, acrylic fibers, cuprammonium rayon, regenerated protein fibers etc. Preferably, the natural fibers or modified natural fibers are selected from the group consisting of cellulose fibers (also referred to as pulp or airfelt) or modified cellulose fibers, such as intra-fiber crosslinked cellulose fibers, cotton fibers, bamboo fibers, viscose fibers or mixtures thereof. More preferably, the natural fibers or modified natural fibers are cellulose fibers or modified cellulose fibers. Synthetic fibers may be selected from the group consisting of polyolefins (such as polyethylene, polypropylene or combinations and mixtures thereof), polyethylene terephthalate (PET), co-PET, polylactic acid (PLA), polybutylene succinate (PBS), polyhydroxy alkanoid (PHA), nylon (or polyamide), or mixtures or combinations thereof.

The fibers in a nonwoven web are consolidated by friction, and/or entanglement, and/or cohesion, and/or adhesion, and/or by heat bonding, pressure bonding, or heat and pressure bonding, and/or ultrasonic bond, excluding paper and products which are woven, knitted, tufted, stitch-bonded. The fibers may be staple fibers (e.g. in carded nonwoven webs) or continuous fibers (e.g. in spunbonded or meltblown nonwoven webs).

The nonwoven webs comprised by the absorbent core of the present invention may comprise or may consist of superabsorbent fibers.

Nonwoven webs can be formed by many processes such as meltblowing, spunlaying, solvent spinning, electrospinning, and carding, and the fibers can be consolidated, e.g. by hydroentanglement (in spunlace nonwoven webs), air-through bonding (using hot air that is blown through the fiber layer in the thickness direction), infrared heat, needle-punching, one or more patterns of bonds and bond impressions created through localized compression and/or application of heat or ultrasonic energy, or a combination thereof. The fibers may, alternatively or in addition, be consolidated by use of a binder. The binder may be provided in the form of binder fibers or particles (which are subsequently molten) or may be provided in liquid, such as a styrene butadiene latex binder. A liquid binder is provided to the fibers (e.g. by spraying, printing or foam application) and is subsequently cured to solidify.

The basis weight of nonwoven webs is usually expressed in grams per square meter ($g/m^2$ or gsm). The basis weight of a nonwoven web may be essentially constant throughout the nonwoven web or may be profiled.

The nonwoven web, especially nonwoven webs consisting of or comprising superabsorbent fibers, may be carded webs formed by needle-punching. In needle punching, the fibers cohesion and the interlacing of the fibers with one another is obtained by means of needles passing through a moving fibrous layer and causing the fibers to intermingle with one another. One or more, or all of the nonwoven webs of the absorbent core of the present invention may also be formed of two or more precursor webs, which are combined with each other by a needle punching process.

Alternatively, one or more, or all of the nonwoven webs of the absorbent core of the present invention may be formed by spunlacing. In a spunlace nonwoven web the fibers have been carded as precursor web and then subjected to hydroentanglement to intermingle and intertwine the fibers with each other. Cohesion and the interlacing of the fibers with one another may be obtained by means of a plurality of jets of water under pressure passing through a moving fleece or cloth and, like needles, causing the fibers to intermingle with one another (hereinafter also referred to as "hydraulic interlacing"). Thus, consolidation of a spunlace nonwoven web is essentially a result of hydraulic interlacing. "Spunlace nonwoven web", as used herein, also relates to a nonwoven web formed of two or more precursor webs, which are combined with each other by hydraulic interlacing.

The two or more webs, prior to being combined into one nonwoven by needle-punching or hydraulic interlacing, may have undergone bonding processes, such as heat and/or pressure bonding by using e.g. a patterned calendar roll and an anvil roll to impart a bonding pattern. However, the two or more webs are combined with each other solely by needle-punching or hydraulic interlacing.

Alternatively, the carded nonwoven web made by needle-punching or spunlacing is a single nonwoven web, i.e., it is not formed of two or more precursor webs. Still in another alternative, one or more, or all of the nonwoven webs of the absorbent core of the present invention may be formed of one precursor web onto which staple fibers are laid down. The staple fibers may be superabsorbent fibers or may comprise superabsorbent fibers. The staple fibers may not have been consolidated into a self-sustaining precursor web but the fibers are loosely laid onto the precursor web. The relatively loose staple fibers are then integrated and intertwined with each other and with the fibers of the underlying precursor web by (only) needle-punching or (only) hydraulic interlacing. Spunlace and/or needle punched nonwoven layers/webs can be made of staple fibers or continuous fibers (continuous fibers are also often referred to as filaments).

Through-air bonding (interchangeably used with the term "air-through bonding") means a process of bonding staple fibers or continuous fibers by forcing air through the nonwoven web, wherein the air is sufficiently hot to melt (or at least partly melt, or melt to a state where the fiber surface becomes sufficiently tacky) the polymer of a fiber or, if the fibers are multicomponent fibers, wherein the air is sufficiently hot to melt (or at least partly melt, or melt to a state where the fiber surface becomes sufficiently tacky) one of the polymers of which the fibers of the nonwoven web are made. The melting and re-solidification of the polymer provide the bonding between different fibers.

"Comprise," "comprising," and "comprises" are open ended terms, each specifies the presence of the feature that follows, e.g., a component, but does not preclude the presence of other features, e.g., elements, steps, components known in the art or disclosed herein. These terms based on the verb "comprise" encompasses the narrower terms "consisting essentially of" which excludes any element, step or ingredient not mentioned which materially affect the way the feature performs its function, and the term "consisting of" which excludes any element, step, or ingredient not specified.

"Body-facing" (also referred to as "skin-facing" herein) and "garment-facing" refer respectively to the relative location of an element or a surface of an element, layer, component. "Body-facing" implies the element or surface is nearer to the wearer during wear than another element of the same component. An example is the absorbent core having a body-facing surface, which is the surface of the absorbent core that is nearer to the body of the wearer than the opposite surface of the absorbent core, which is garment-facing. "Garment-facing" implies the element or a surface of an element, layer, component, is more remote from the wearer during wear than another element or a surface of an element, layer, component, or of the absorbent article as a whole. The garment-facing surface may face another (i.e. other than the wearable article) garment of the wearer, other items, such as the bedding, or the atmosphere. Typically, the body-facing surface of the topsheet forms a least a portion of the body-facing surface of the absorbent article as a whole, and the garment-facing surface of the outer cover nonwoven forms at least a portion of the garment-facing surface of the absorbent article as a whole.

The "wearer's skin" as used herein refers to those parts of the skin which are covered by the absorbent article. In the test method below, the collagen is used to represent the wearer's skin.

<u>Absorbent article comprising an acquisition and distribution system comprising an acquisition and/ or distribution layer</u>

Skin health and protection from biological insults are important for wearers of absorbent articles 10. Absorbent articles 10 such as diapers, pants, or adult incontinence products, are worn such that they are in direct contact with the skin of the wearer. An unavoidable consequence of the use of absorbent articles 10 is that the skin is exposed more directly to various physical and biological insults. Consequently, the barrier function of the skin covered by the absorbent article 10 is put at risk.

The decay of urea, which has entered the absorbent article 10, such as a diaper or pant, leads to the formation of ammonia. It has been found that ammonia elevates the skin pH. The pH value of skin should be between 4.0 and 6.0, or optimally between 4.5 and 5.5. Hence, skin's natural pH is mildly acidic. This mildly acidic pH is created by the skin's acid mantle, the water part of the hydrolipid film that protects the external layers of skin.

Skin's pH has been found to play an important role in skin condition. The acid mantle is key to skin's protective barrier. For example, it inhibits the growth of bacteria and restores and maintains the optimal acid environment in which skin's natural flora can thrive. If skin's pH rises into the alkaline range, its natural balance is disturbed. In this condition, the outer layer of skin (or epidermis) is no longer able to work as a protective barrier.

When skin's barrier function is compromised it is less resilient and more sensitive to environmental triggers. It can become dry, sensitive or hypersensitive, and so-called diaper rash can occur if this happens in the area of the skin which is covered by the absorbent article 10. It is thus very desirable to maintain the pH of the skin that comes into contact with the absorbent article 10, within the range of pH 4.0 to 6.0. Given that it has been found that elevated levels of ammonia contribute to an increase of skin pH, one way of helping the maintenance of the mildly acidic skin pH, is to maintain low ammonia levels inside the diaper. On the other hand, acidic compounds may decrease the skin pH to an undesired level of below 4.5 as well. Consequently, it is desirable to neutralize ammonia produced while not exposing the skin to acidic components by e.g. applying it close to the wearer's skin, such as by incorporating an acidic compound in a lotion that is applied on the body-facing surface of the topsheet 26, or by otherwise providing an acidic compound on the body-facing surface of the topsheet 26.

The inventors have found that providing the acidic compounds in the acquisition and distribution system 38 is particularly suited to prevent free ammonia in the diaper and thus improves skin health. The acidic compound is provided in such amounts that an acquisition and/ or distribution layer of the acquisition and distribution system 38 exhibits an Acid Equivalent value of from 0.050 mmol/g to 6.000 mmol/g, preferably from 0.100 mmol/g to 3.000 mmol/g, more preferably from 0.150 mmol/g to 2.000 mmol/g, even more preferably from 0.180 mmol/g to 1.000 mmol/g, even more preferably from 0.200 mmol/g to 0.850 mmol/g, or even from 0.220 mmol/g to 0.500 mmol/g according to the Acid Equivalent method disclosed herein. Having an Acid Equivalent values of the acquisition and/ or distribution layer in the ranges described ensures that ammonia formation and free ammonia can be reduced while skin health is not negatively affected by the acidic components.

The acidic compound may be provided in such amounts that the acquisition and/ or distribution layer may exhibit an Acid Equivalent value of 0.050 mmol/g or higher, preferably 0.100 mmol/g or higher, more preferably 0.150 mmol/g or higher, even more preferably 0.200 mmol/g or higher, or even 0.220 mmol/g or higher according to the Acid Equivalent method disclosed herein.

In another form, the acquisition and distribution system 38 may comprise an acquisition and/or distribution material that exhibits an Acid Equivalent value of from 0.050 mmol/g to 6.000 mmol/g, preferably from 0.100 mmol/g to 3.000 mmol/g, more preferably from 0.150 mmol/g to 2.000 mmol/g, even more preferably from 0.180 mmol/g to 1.000 mmol/g, even more preferably from 0.200 mmol/g to 0.850 mmol/g, or even from 0.220 mmol/g to 0.500 mmol/g according to the Acid Equivalent method disclosed herein. The acquisition and/or distribution material may be provided in such amounts that at least one acquisition and/or distribution layer of the acquisition and distribution system 38 may exhibit an Acid Equivalent value of from 0.050 mmol/g to 6.000 mmol/g, preferably from 0.100 mmol/g to 3.000 mmol/g, more preferably from 0.150 mmol/g to 2.000 mmol/g, even more preferably from 0.180 mmol/g to 1.000 mmol/g, even more preferably from 0.200 mmol/g to 0.850 mmol/g, or even from 0.220 mmol/g to 0.500 mmol/g according to the Acid Equivalent method disclosed herein. The acquisition and/or distribution layer may comprise from 50% to 100%, from 60% to 99.5%, from 70% to 99%, from 80% to 98%, from 90% to 95% by weight of the acquisition and/or distribution layer of an acquisition and/or distribution material exhibiting the described Acid Equivalent value. The acquisition and distribution system 38 may comprise from 50% to 100%, from 60% to 99.5%, from 70% to 99%, from 80% to 98%, from 90% to 95% by weight of the acquisition and distribution system 38 of an acquisition and/or distribution material exhibiting the described Acid Equivalent value.

The Acid Equivalent values may be achieved by providing one or more acids in the acquisition and/ or distribution layer of the acquisition and distribution system 38, the acquisition and/or distribution material respectively. The one or more acids may be or preferably may each be monomeric acids. The one or more monomeric acids may be or preferably may each be a monomeric organic acid. The one or more monomeric acids may be or preferably may each be selected from the group consisting of carboxylic acids, amino acids and sulfonic acids. The one or more monomeric acids may be or preferably may each be monomeric carboxylic acids. The one or more monomeric acids may be or preferably may each be diprotonic and/or triprotonic acids. The one or more monomeric acids may be or preferably may each be non-ethylenical acids, in particular non-ethylenical carboxylic acids.

The acquisition and/or distribution material may comprise one or more non-ethylenical acids. Non-ethylenical acids means that the carboxyl carbon atom is not directly bond to an ethylene group, i.e. that there is no conjugated pi electron system between the carboxyl carbon atom and the directly neighbouring carbon atoms. In contrast to non-ethylenical acids, acids such as acrylic acid, methacrylic acid or crotonic acid are ethylenical acids.

In one form at least one of the carboxylic acids may be selected from the group of saturated monocarboxylic acids, dicarboxylic acids or tricarboxylic acids and may have a p$K_a$ for the first acid dissociation in water at 25°C and 1 atm from 2.5 to 5.0. This is due to them being strong enough to neutralize ammonia, while not being too strong to potentially harm the wearer's skin, in case the skin would be exposed to the acid. The $pK_a$ at 25°C and 1 atm can be obtained from the literature. In case of doubt, $pK_a$ values from Dawson, R.M.C. et al., Data for Biochemical Research, Oxford, Clarendon Press, 1959 rounded to 0.1 are to be used. If the $pK_a$ value for a carboxylic acid is not given in Dawson, R.M.C. et al., Brown, H.C. et al., the $pK_a$ values given in Braude, E.A. and F.C. Nachod, Determination of Organic Structures by Physical Methods, Academic Press, New York, 1955 rounded to 0.1 are to be used. Examples of carboxylic acids with a $pK_a$ for the first acid dissociation in water at standard temperature 25°C and 1 atm from 2.5 to 5.0 are: acetoacetic ($pK_a$ = 3.6), acetopyruvic (2.6), trans-acotinic (2.8), citric (3.1), formic (3.8), fumaric (3.0), glyceric (3.5), glycollic (3.8), glyoxylic (3.3), lactic (3.9), malic (3.4), L-tartaric (2.9), meso-tartaric (3.2), vinylacetic (4.4), malonic (2.8) or succinic acid (4.2).

The acquisition and/ or distribution layer of the acquisition and distribution system 38 may comprise malic acid. Malic acid beneficially inhibits antimicrobial growth and is employed as antioxidant in skin care applications, thus providing additional healthcare benefits. The malic acid may be naturally sourced. The malic acid may be bio-based. In particular, malic acid may be present only in L-form. It has surprisingly been found that malic acid is effective in neutralizing ammonia and keeping the skin pH in a desired range even when employing very low amounts.

The acquisition and distribution system 38 and/ or the acquisition and/ or distribution layer may comprise, acrylic acid, oligoacrylic acid, polyacrylic acid and/ or salts thereof. Acrylic acid, oligoacrylic acid, polyacrylic acid, potentially in their partially neutralized form as salts, are typically present in crosslinked cellulose or due to the acquisition and distribution system 38 and/ or the acquisition and/ or distribution layer being in contact with the core and the contained SAP material. The acquisition and distribution system 38 and/ or the acquisition and/ or distribution layer may be substantially free of citric acid.

In particular, the acquisition and distribution system 38 and/ or acquisition and/ or distribution layer may comprise substantially no free acids except malic acid, acrylic acid, oligoacrylic acid, polyacrylic acid or combinations thereof or salts thereof. Substantially in this context means that free acids - other than malic acid, acrylic acid, oligoacrylic acid, polyacrylic acid or combinations thereof or salts thereof - are present in at most 2% by weight of the total amount of free acids. Acrylic acid, oligoacrylic acid and polyacrylic acid may be present in an amount that is less than a total of 50%, preferably less than a total of 25%, more preferably less than a total of 10% by weight of the total amount of free acids.

Free acids are acids that are at least partially, in particular about at least 10 g/l (at 20°C), soluble (dissolvable) in water. Further, free acids may not be covalently bound to the acquisition and/ or distribution layer. In particular, free acids may not be chemically bound to the acquisition and/ or distribution layer. Thus, no covalent or ionic bonds may be present between the free acids and the acquisition and/ or distribution layer.

While application of monomeric acids on the topsheet 26 of an absorbent article 10 is known, e.g., where a monomeric carboxylic acid such as lactic acid is applied in a lotion on a topsheet 26, the amounts of these compounds in the lotion are typically very low. Such low amounts may not be sufficient to obtain the desired result, namely the maintenance of the skin pH in the desired range. It may become challenging to incorporate, e.g. in the lotion, monomeric acids in amounts required to neutralize ammonia sufficiently to maintain the pH of the wearer's skin in the desired range. Moreover, lotion provided on the topsheet 26 of an absorbent article 10, is typically hydrophobic and contains very little to no water. The acid may thus be "trapped" in the lotion which may limit its ability to reduce the ammonia amount in the absorbent article on decay of urea. Hence, also higher amounts of acid may not be sufficient to maintain the pH of the wearer's skin in the desired range. Applying acids as part of the acquisition and distribution system 38 and additionally in a form that maximizes its solubility in urine, thus providing the acquisition and/ or distribution layer with Acid Equivalent values described herein, makes it much easier to provide acids in amounts where it can actually contribute to the maintenance of the skin pH in a desired range.

While urine and feces initially get into contact with the body-facing surface of the topsheet 26, it has been found that there is no need to provide the one or more acids on the topsheet 26 to be able to be immersed in the urine. Instead, providing one or more acids deeper inside the absorbent article 10 in the acquisition and distribution system 38 and thus underneath the topsheet 26, positions the one or more monomeric acids close to the location where the urine gets ultimately stored (typically within the absorbent core 30) has proven beneficial. Further, with the acquisition and distribution system 38 being located between the topsheet 26 and the absorbent core 30, the one or more acids are prevented from getting in contact with the wearer's skin but are located between the absorbent core 30 and the wearer's skin such that ammonia, either in free or dissolved form, has to pass by the location of the one or more acids before reaching the wearer's skin and thus can be neutralized. Further, providing the one or more acids in the acquisition and distribution system 38 and thus in layers participating in the fluid transfer between the topsheet 26 and the absorbent core 30 facilitates solvation (dissolution) of the one or more acids in urine and thus enhances the activity.

In one form, the acquisition and distribution system 38 may comprise one or more monomeric acids and the one or more monomeric acids are distributed throughout the at least one acquisition and/ or distribution layer. Distributed throughout in this context means that acquisition and/ or distribution material obtained from any part of the at least one acquisition and/ or distribution layer exhibits an Acid Equivalent value as acquisition and/ or distribution material from any other part of this same acquisition and/ or distribution layer with a difference of not more than 0.050 mmol/g, wherein the any parts are not less than 100 mg. Preferably, the acquisition and/ or distribution material comprised by the at least one acquisition and/ or distribution layer may exhibit an Acid Equivalent value of from 0.050 mmol/g to 6.000 mmol/g, preferably from 0.100 mmol/g to 3.000 mmol/g, more preferably from 0.150 mmol/g to 2.000 mmol/g, even more preferably from 0.180 mmol/g to 1.000 mmol/g, even more preferably from 0.200 mmol/g to 0.850 mmol/g, or even from 0.220 mmol/g to 0.500 mmol/g according to the Acid Equivalent method disclosed herein. Such a distribution ensures that ammonia is neutralized no matter the distribution of the urine in the layer. The one or more monomeric acids may be distributed throughout the at least one acquisition and/ or distribution layer by applying the one or more monomeric acids to at least part of the fibers forming the at least one acquisition and/ or distribution layer prior to or during forming the at least one acquisition and/ or distribution layer. Due to the application of the acids to the fibers prior to or during forming the layer, a more uniform distribution can be achieved.

The acquisition and/or distribution layer has a garment-facing surface and a body-facing surface. The garment-facing surface and a body-facing surface may have a substantially same/equivalent level of acid per area. Such level of acid per area may be determined by contacting the surface areas with pre-wetted pH paper or pH indicator stripes, or via a suitable surface pH meter with a suitable surface pH electrode. Differences in pH of 0.5 units or more between the garment-facing surface and a body-facing surface are considered different in the level of acid per area.

The acquisition and distribution system 38 may comprise natural fibers, and/or comprises cellulose-based fibers. The one or more monomeric acids may be applied to the natural fibers, and/or cellulose-based fibers prior to or during forming the acquisition and distribution system 38 and/or acquisition and/ or distribution layer.

The acquisition and distribution system 38 may comprise a nonwoven layer, preferably a carded nonwoven layer, more preferably a carded nonwoven comprising PET staple fibers.

The acquisition and distribution system 38 may comprise one or more acquisition and/or distribution layers with a basis weight of about 40 gsm or less, preferably about 35 gsm or less. The acquisition and distribution system 38 may comprise one or more acquisition and/or distribution layers with a basis weight of about 10 gsm or more, or preferably about 15 gsm or more, or in the range between 10 to 40 gsm, or more preferably in the range between 15 to 35 gsm. The one or more acquisition and/or distribution layers may correspond to the acquisition and/or distribution layer exhibiting the Acid Equivalence value described herein, or may be different from the acquisition and/or distribution layer exhibiting the Acid Equivalence value, the acquisition and/or distribution layer comprising the acquisition and/or distribution material exhibiting the Acid Equivalence value respectively.

Moreover, providing the one or more acids away from the skin-contacting surface of the absorbent article 10, eliminates or largely reduces the amount of the one or more monomeric acids which gets into direct contact with the skin of the wearer, thus reducing any potential risk of lowering the skin pH or adversely affecting the skin barrier layer. Reducing the number of different substances and compounds that are transferred to skin, may be appreciated by users or caregivers, as exposing the skin to a large variety of different substances may be perceived stressful for the skin by the user or caregiver. The acquisition and distribution system 38 and/ or the acquisition and/or distribution layer may comprise at most 4 different monomeric, non-ethylenical free acids, preferably at most 3 different monomeric , non-ethylenical free acids, more preferably at most 2 different monomeric, non-ethylenical free acids or even only one free monomeric, non-ethylenical acid. The acquisition and distribution system 38 and/ or the acquisition and/or distribution layer may comprise at most 4 different monomeric free acids, preferably at most 3 different monomeric free acids, more preferably at most 2 different monomeric free acids or even only one free monomeric acid. The acquisition and distribution system 38 and/ or the acquisition and/or distribution layer may comprise at most 4 different free acids, preferably at most 3 different free acids, more preferably at most 2 different free acids or even only one free acid.

The absorbent article 10 comprises a liquid permeable topsheet 26, a liquid impermeable backsheet 28, an absorbent core 30, and an acquisition and distribution system 38 comprising at least one acquisition and/ or distribution layer. The absorbent core 30 is provided between the topsheet 26 and the backsheet 28. The acquisition and distribution system 38 is provided between the topsheet 26 and the absorbent core 30. The acquisition and/ or distribution layer is comprised by the acquisition and distribution system 38 and may contribute to the acquisition, the distribution or both the acquisition and distribution of bodily exudates.

It has surprisingly been found that relatively low quantities of acids are sufficient to effectively neutralize ammonia in the diaper and thus maintain the skin pH of the wearer in the desired range. It is sufficient for the acquisition and/ or distribution layer of the acquisition and distribution system 38 to exhibit an Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein.

The one or more monomeric acids each may be partially present in unreacted form. Further, the one or more monomeric acids each may not be covalently bound to the acquisition and/ or distribution layer. In particular, the one or more monomeric acids each may not be chemically bound to the acquisition and/ or distribution layer. Thus, no covalent or ionic bonds may be present between the one or more monomeric acids and the acquisition and/ or distribution layer. Hence, the one or more monomeric acids may readily be dissolved in urine or other body fluids.

The one or more monomeric acids each may not form part of a lotion. As discussed, being part of a lotion may reduce the acid availability and activity. The one or more monomeric acids each may not form part of a buffer system. Further, the acquisition and distribution system 38 may comprise no salt of any of the one or more monomeric acids.

The absorbent core 30 may comprise at least 5.0 g of superabsorbent polymer material. In particular, the absorbent core 30 may comprise from 5.0 g to 20.0 g, preferably from 7.5 g to 15.0 g, or even from 10.0 g to 12.5 g of superabsorbent polymer material. Due to its acidity, the superabsorbent polymer material may beneficially contribute to the neutralization of ammonia. However, the amounts of SAP material should not be too high to avoid migration of the SAP material within the core which could compromise liquid handling and comfort performance of the article.

The acquisition and distribution system 38 may comprise at least two acquisition and/ or distribution layers. A first layer of the acquisition and distribution system 38 may comprise a nonwoven material and a second layer of the acquisition and distribution system may comprise a cross-linked cellulosic material. The first layer of the acquisition and distribution system 38 may comprise a carded nonwoven material. The second layer of the acquisition and distribution system may be provided between the first layer of the acquisition and distribution system and the absorbent core. The first layer of the acquisition and distribution system may be provided between the topsheet and the second layer of the acquisition and distribution system.

The first layer of the acquisition and distribution system 38 may comprise a nonwoven with a basis weight of about 40 gsm or less, or preferably about 35 gsm or less. The first layer of the acquisition and distribution system 38 may comprise a nonwoven with a basis weight of about 10 gsm or more, or preferably about 15 gsm or more, or in the range between 10 to 40 gsm, or more preferably in the range between 15 to 35 gsm.

The second layer of the acquisition and distribution system 38 may comprise a cross-linked cellulosic material and may have a basis weight of at least 100 gsm, or preferably of at least 150 gsm, or of at least 180 gsm, or in the range between 150 to 250 gsm, or preferably in the range between 180 to 230 gsm.

The first layer of the acquisition and distribution system 38 may exhibit an Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein. Preferably, the second layer of the acquisition and distribution system 38 may exhibit an Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein.

The one or more monomeric acids may be distributed throughout the second acquisition and/ or distribution layer. The one or more monomeric acids may be distributed throughout the second acquisition and/ or distribution layer by applying the one or more monomeric acids after and/ or during the step of crosslinking the cellulosic material. The one or more monomeric acids may be applied to at least part of the fibers forming the second acquisition and/ or distribution layer prior to or during forming the second acquisition and/ or distribution layer. Due to this application a more uniform distribution can be achieved, and the acids do not interfere with or participate in the crosslinking reaction. Further, bonding of the acids with the fibers may be prevented.

In one aspect, the second acquisition and/ or distribution layer may comprise the one or more monomeric acids, while the first acquisition and/ or distribution layer is substantially free of monomeric acid. Due to such distribution the likelihood of acid being transferred to the skin is minimized.

The acquisition and distribution system 38 and/ or the comprised acquisition and distribution layers may have $pH_0$ value of about 5.5 or lower, or of about 5.0 or lower, or of about 4.5 or lower, or of about 4.0 or lower according to the $pH_0$ Test Method disclosed herein. The acquisition and distribution system 38 and/ or the comprised acquisition and distribution layers may have a $pH_0$ value of about 2.5 or higher, or of about 3.0 or higher, or from 3.0 to 5.5, from 3.5 to 5.0 according to the $pH_0$ Test Method disclosed herein.

The acquisition and distribution system 38 and/ or the comprised acquisition and distribution layers may have conductivity value of about 0.05 mS/cm or higher, or of about 0.1 mS/cm or higher, or between 0.1 mS/cm and 0.2 mS/cm according to the Conductivity Test Method disclosed herein.

The liquid permeable topsheet 26 has a first surface which corresponds to a body-facing surface in the absorbent article 10, and a second surface opposing the first surface, which corresponds to a garment-facing surface in the absorbent article 10. The body-facing, i.e. the first, surface may be in direct contact with the skin of the wearer of the absorbent article 10.

The absorbent core 30 has a first surface, which corresponds to the body-facing surface in the assembled absorbent article 10, and a second surface opposing the first surface, which corresponds to a garment-facing surface in the assembled absorbent article 10. The garment-facing second surface of the absorbent core 30 may be in direct contact with the backsheet 28 in the absorbent article 10.

It is known that under anaerobic conditions certain bacteria use the denitrification process to generate energy. Specifically, they use nitrates (e.g., derived from ammonia) to oxidate glucose as their metabolic pathway for energy generation. Hence, it has been found that, to reduce ammonia production in an absorbent article 10 during use, having an absorbent structure with high permeability to promote aerobic conditions inside the absorbent article 10 can be beneficial in addition to the use of one or more monomeric acids. In one aspect of the present invention, the provided absorbent core 30 thus has a permeability of from $10^{-6}$ cm$^2$ to $10^{-4}$ cm$^2$ according to the IPRP test method set out herein. Good permeability can e.g., be achieved by providing the absorbent core 30 with regions of reduced caliper, such as elongated "channels" within the absorbent core 30, where little or no absorbent material is provided.

The acquisition and distribution system 38 has a first surface, which corresponds to a body-facing surface in the absorbent article 10, and a second surface opposing the first surface, which corresponds to a garment-facing surface in the absorbent article 10. The acquisition and distribution system 38 may comprise or consist of a first layer and a second layer (hence, there may be at least two acquisition and/ or distribution layers). Analogously the first and second acquisition and/ or distribution layers each have a first surface, which corresponds to a body-facing surface in the absorbent article 10, and a second surface opposing the first surface, which corresponds to a garment-facing surface in the absorbent article 10. Nowhere may the acquisition and distribution system 38 and any layer thereof be longer than the absorbent core 30 along the longitudinal dimension of the absorbent article 10. Also, nowhere may acquisition and distribution system 38 and any layer thereof be wider than the absorbent core 30 along the transverse dimension of the absorbent article 10.

The acquisition and distribution system 38 may comprise an acquisition and/or distribution layer having a body-facing surface and an opposing garment-facing surface; the layer has a surface pH value of the body-facing surface (pH_bfs) and a surface pH value of the garment-facing surface (pH_gfs), wherein the pH_bfs and the pH_gfs differ by at most 0.2, preferably by at most 0.1, or more preferably by at most 0.05 according to the Surface pH Test Method as set out herein.

The absorbent core 30 of the absorbent article 10 may have a permeability of from $10^{-6}$ cm$^2$ to $10^{-4}$ cm$^2$ according to the IPRP test method set out herein. Further, the absorbent core 30 has elongated regions with reduced caliper.

During use of an absorbent article 10, a part of the urine which has been transferred through the topsheet 26 into the absorbent article 10, may penetrate back through the topsheet 26 before it can be absorbed and stored by the absorbent core 30. This is also known as "rewet". However, if one or more monomeric acids are applied underneath the topsheet 26, at least a part of the acids may be "washed out" of the absorbent article 10 by being dissolved in the urine which then penetrates back through the topsheet 26 onto the body-facing surface of the absorbent article 10. To reduce the amount of one or more monomeric acids which may be transported onto the wearer-facing surface of the absorbent article 10 during use, it is desirable that the absorbent article 10 has good rewet properties. Thus, the absorbent article 10 may have a rewet of less than 150 mg, or less than 120 mg according to the Post Acquisition Collagen Rewet Test Method set out herein.

Further, a process for making an absorbent article 10 comprising a liquid permeable topsheet 26, a liquid impermeable backsheet 28, an absorbent core 30, and an acquisition and distribution system 38 comprising an acquisition and/ or distribution layer is provided. The absorbent core 30 is provided between the topsheet 26 and the backsheet 28 and the acquisition and distribution system 38 is provided between the topsheet 26 and the absorbent core. The process comprises the step of

a) applying one or more monomeric acids to an acquisition and/ or distribution layer in an amount such that the acquisition and/ or distribution layer exhibits an Acid Equivalent value of from 0.050 mmol/g to 6.000 mmol/g, preferably from 0.100 mmol/g to 3.000 mmol/g, more preferably from 0.150 mmol/g to 2.000 mmol/g, even more preferably from 0.180 mmol/g to 1.000 mmol/g, even more preferably from 0.200 mmol/g to 0.850 mmol/g, or even from 0.220 mmol/g to 0.500 mmol/g according to the Acid Equivalent method disclosed herein.

The one or more monomeric acids each may be applied to the acquisition and/ or distribution layer before the acquisition and distribution system 38 is provided at the manufacturing line of the absorbent article 10. The one or more monomeric acids each may be applied to the acquisition and/ or distribution layer or to materials comprised in the acquisition and/ or distribution layer before the acquisition and/ or distribution layer or to materials comprised in the acquisition and/ or distribution layer are provided at the absorbent article manufacturing line. Such a sequence minimizes the adaptations to existing manufacturing lines and potentially increases the speed of manufacture. Alternatively, the one or more monomeric acids each may be applied to the acquisition and/ or distribution layer at the manufacturing line. As anticipated by the skilled person, the absorbent article 10 may be partially assembled, when the one or more monomeric acids each are applied to the acquisition and/ or distribution layer.

The one or more monomeric acids each may be applied to the acquisition and/ or distribution layer in solution and/ or suspension. In particular, the one or more monomeric acids may be applied to the acquisition and/ or distribution layer in one solution and/ or suspension. Alternatively, the one or more monomeric acids may be applied to the acquisition and/ or distribution layer in one solution and/ or suspension each. Preferably, one or more monomeric acids each are applied to the acquisition and/ or distribution layer in an aqueous solution and/ or suspension.

The one or more monomeric acids may be applied to at least part of the fibers forming at the acquisition and/ or distribution layer of the acquisition and distribution system 38 prior to the acquisition and/ or distribution layer being formed. An acquisition and/ or distribution layer may comprise cross-linked cellulosic material. The one or more monomeric acids may be distributed throughout the acquisition and/ or distribution layer by applying the one or more monomeric acids after and/ or during the step of crosslinking the cellulosic material.

The solvent may be any liquid substance capable of at least partially dissolving the one or more monomeric acids. It is desirable that the solvent does not contain any liquids potentially harmful to humans. Preferred solvents are non-toxic alcohols, such as ethanol or glycerol, and water. Alcohols or alcohol/ water combinations may be removed at less harsh conditions. Nevertheless, water, in particular deionized and/ or distilled water, is preferably used to obtain an aqueous solution. Hence, it can be avoided that harmful substances and/ or substances that may in any way influence the performance of the absorbent article 10 are introduced by the solvent.

The solution comprising the one or more monomeric acids may be applied by any means known to the skilled person. Suitable methods include spray- and/ or dip-coating and/ or slot-coating. For applying it to a surface, e.g. of the layer of the acquisition and distribution system 38, the solution may be applied by spraying or by employing a so-called kiss role. When the solution is applied to a whole layer of the acquisition and distribution system 38, the layer may be dipped or dragged through an immersion bath. Alternatively, the one or more monomeric acids may be suspended in a polyol and then applied to the acquisition and/ or distribution layer. In particular, the one or more monomeric acids may be dispersed in glycerol as fine particles and then spray coated onto the acquisition and/ or distribution layer. Both nozzles for spraying and immersion baths may be easily integrated in manufacturing lines for absorbent articles 10 or the layer of the acquisition and distribution system 38 may be treated with a solution or dispersion prior to being provided to a manufacturing line.

The solvent may be removed prior to assembling the absorbent article. In particular, the solvent may be removed at temperatures of from 20°C to 100°C, preferably from 25°C to 80°C, more preferably from 30°C to 60°C or even from 40°C to 50°C. Additionally, the solvent may be removed at reduced pressure. This means that the pressure is less than atmospheric pressure. This can be achieved e.g. by applying vacuum means. The temperature, which the acquisition and distribution system 38 is exposed to, may not exceed 100 °C, preferably not exceed 80°C, more preferably not exceed 60°C, even more preferably not exceed 40°C or even not exceed 30°C during the process. Temperatures exceeding the mentioned limits should be avoided to prevent chemical reaction and/ or damages to the absorbent article 10.

The one or more monomeric acids each may be applied to the acquisition and/ or distribution layer in solution and the solvent may be removed prior to providing the acquisition and/ or distribution layer/ the acquisition and distribution system 38 at a manufacturing line. Thus, contaminations due to the solvent and damages to solvent-prone parts of the manufacturing lines may be avoided. After applying in solution and subsequent drying, the one or more monomeric acids are in their immobilized solid form, and thus it is further avoided that - before use - one or more monomeric acids penetrates to and/or interacts with parts of the absorbent article, it was not applied to.

The acquisition and distribution system 38 and/ or the comprised acquisition and distribution layers are exposed to a heating and/or drying step for not more than 7 hours during the process, or not subjected to any heating and/or drying step at all after application of the one or more monomeric acids. In this context heating step means a step in which the temperature is raised above 50°C.

Alternatively, the acquisition and distribution system 38 may be placed on the absorbent core 30 and the one or more monomeric acids each may be applied to the acquisition and distribution system 38 in an aqueous solution and/ or suspension afterwards. In addition, the one or more monomeric acids may be dissolved and/ or suspended in less than 500 ml, preferably less than 400 ml, more preferably less than 300 ml or even more preferably less than 200 ml, less than 100 ml or even less than 50 ml of water per absorbent article. By this, the absorbency of the SAP material in the absorbent article 10 is not reduced significantly although the water of the monomeric acid solution may be absorbed by the SAP material.

Absorbent articles in general

The disclosure of absorbent articles herein below is intended to refer to the aspects of the present invention, which refer to the absorbent article as such, as well as to the method of making an absorbent article, i.e. the disclosure is meant to describe aspects of the respective absorbent article. While the one or more monomeric acids or their application is not specifically described in the following disclosure of absorbent articles and the one or more monomeric acids is also not shown in the Figures, it goes without saying that the absorbent articles of the present invention describe the one or more monomeric acids as described and claimed herein and in the Examples below.

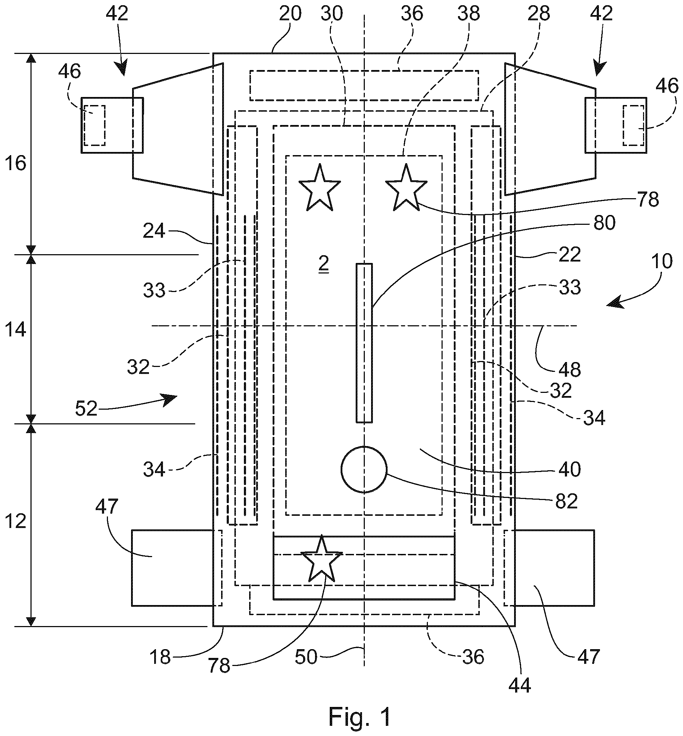

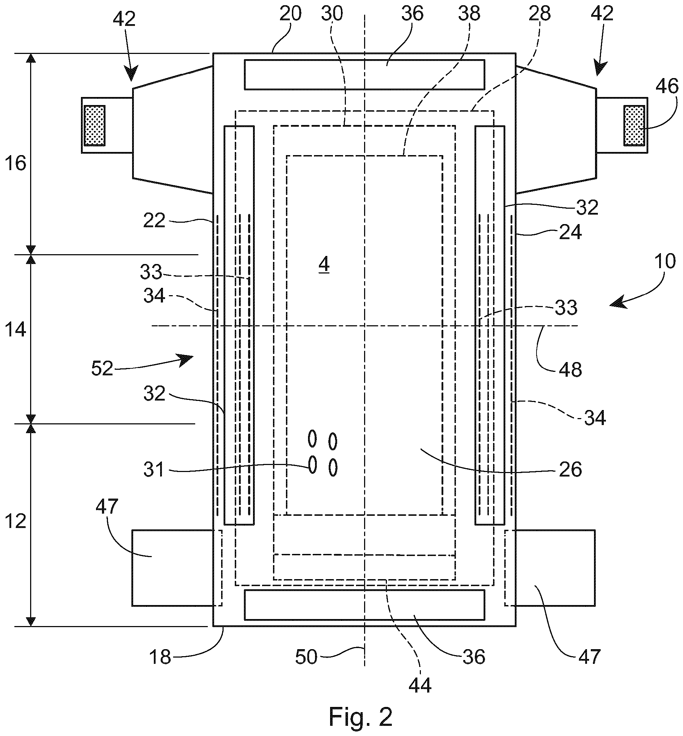

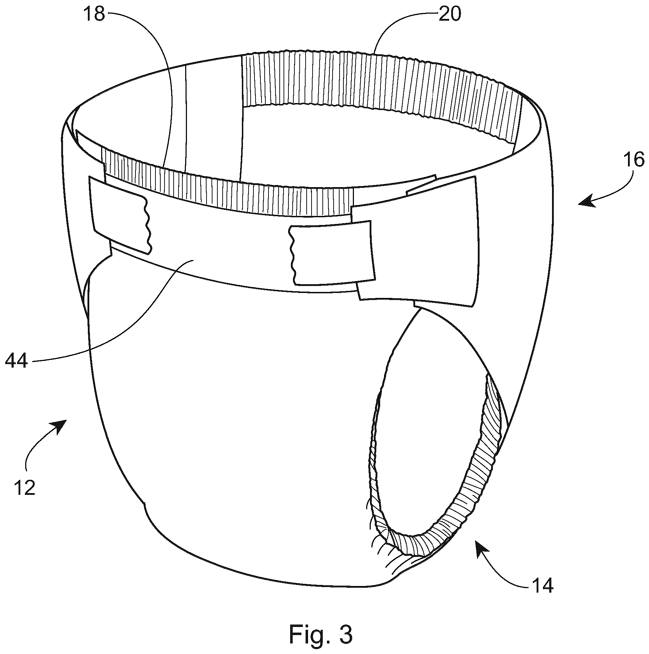

An example absorbent article 10 according to the present disclosure, shown in the form of a diaper (applied on the wearer with tapes), is represented in Figs. 1-3. Fig. 1 is a plan view of the example absorbent article 10, garment-facing surface 2 facing the viewer in a flat, laid-out state (i.e., no elastic contraction). Fig. 2 is a plan view of the example absorbent article 10 of Fig. 1, body-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of diapers, including adult incontinence products, pants, or other absorbent articles, such as sanitary napkins and absorbent pads, for example.

The absorbent article 10 may comprise a front waist region 12, a crotch region 14, and a back waist region 16. The crotch region 14 may extend intermediate the front waist region 12 and the back waist region 16. The front waist region 12, the crotch region 14, and the back waist region 16 each are 1/3 of the length of the absorbent article 10. The absorbent article 10 may comprise a front waist edge 18, a back waist edge 20 opposite to the front waist edge 18. The absorbent article 10 further comprise longitudinally extending, transversely opposed side edges 22 and 24 defined by the chassis 52.

The absorbent article 10 comprises a liquid permeable topsheet 26, a liquid impermeable backsheet 28, and an absorbent core 30 positioned between the topsheet 26 and the backsheet 28. Herein below, the terms "topsheet" and "backsheet" is used to describe a liquid permeable topsheet and a liquid impermeable backsheet.

The absorbent article 10 may also comprise one or more pairs of barrier leg cuffs 32 with or without elastics 33, one or more pairs of leg elastics 34, one or more elastic waistbands 36,

The absorbent article comprises an acquisition and distribution system 38 which is provided in between the topsheet 26 and the absorbent core 30.

An outer cover material 40, such as a nonwoven material, may cover a garment-facing side of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 may have a transverse centerline 48 and a longitudinal centerline 50. The transverse centerline 48 extends perpendicular to the longitudinal centerline 50.

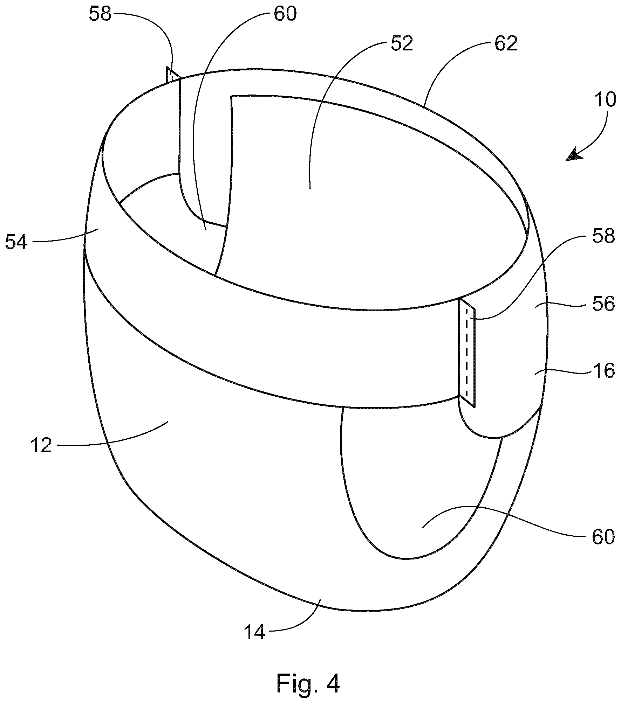

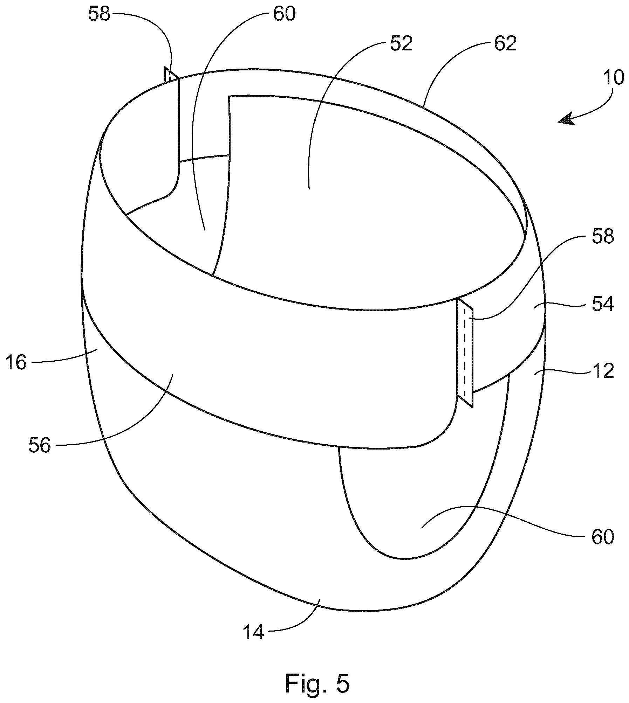

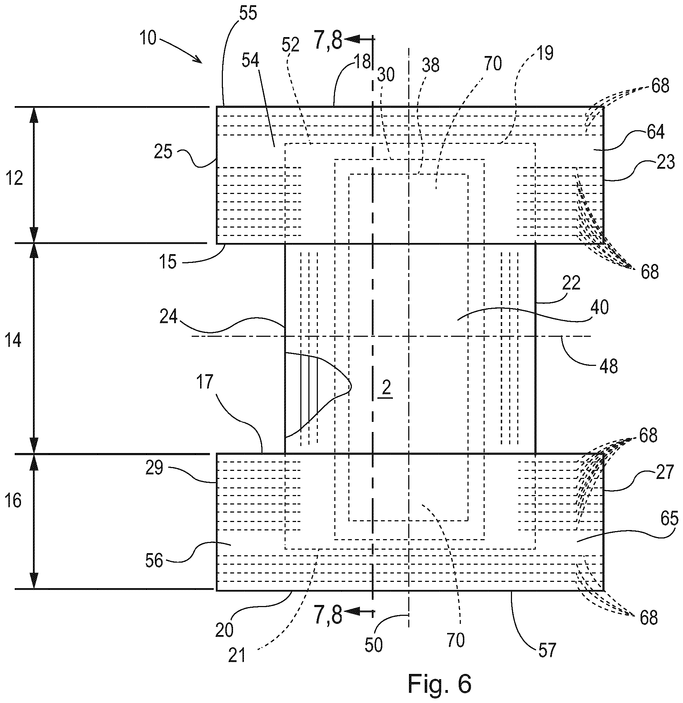

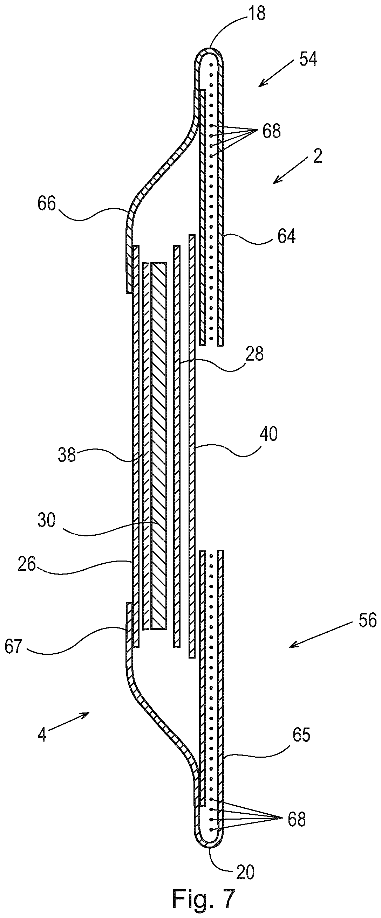

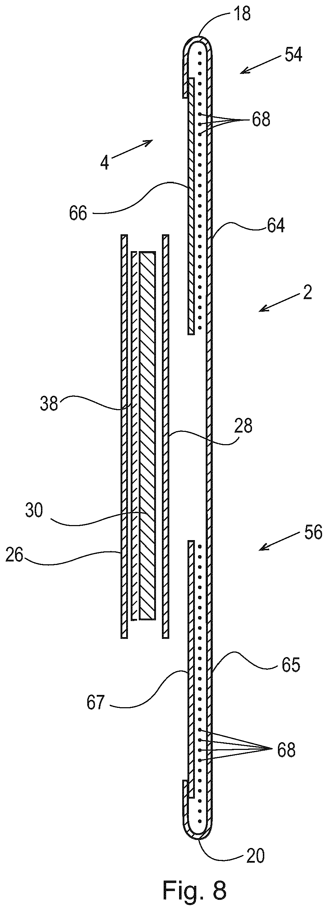

In other instances, the absorbent article may be in the form of a pant having permanent or refastenable side seams. Suitable refastenable seams are disclosed in U.S. Pat. Appl. Pub. No. 2014/0005020 and U.S. Pat. No. 9,421,137. Referring to Figs. 4-8, an example absorbent article 10 in the form of a pant is illustrated. Fig. 4 is a front perspective view of the absorbent article 10. Fig. 5 is a rear perspective view of the absorbent article 10. Fig. 6 is a plan view of the absorbent article 10, laid flat, with the garment-facing surface facing the viewer. Elements of Fig. 4-8 having the same reference number as described above with respect to Figs. 1-3 are the same element (e.g., absorbent core 30). Fig. 7 is an example cross-sectional view of the absorbent article taken about line 7-7 of Fig. 6. Fig. 8 is an example cross-sectional view of the absorbent article taken about line 8-8 of Fig. 6. Figs. 7 and 8 illustrate example forms of front and back belts 54, 56. The absorbent article 10 may have a front waist region 12, a crotch region 14, and a back waist region 16. Each of the regions 12, 14, and 16 may be 1/3 of the length of the absorbent article 10. The absorbent article 10 comprises a topsheet 26, a backsheet 28, and an absorbent core 30 disposed at least partially intermediate the topsheet 26 and the backsheet 28, and at least one acquisition and distribution system 38 which is provided in between the topsheet 26 and the absorbent core, similar to that as described above with respect to Figs. 1-3. The absorbent article 10 may comprise a front belt 54 in the front waist region 12 and a back belt 56 in the back waist region 16. The central chassis 52 (comprising the topsheet 26, backsheet, the absorbent core and the acquisition and distribution system 38 may be joined to a body-facing surface 4 of the front and back belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

In another form, the absorbent article may be an absorbent insert for use with a reusable outer cover. The insert may be disposable or reusable. The reusable outer cover may comprise a woven or other material and may be configured as a pant or a diaper. In the diaper context, the reusable outer cover may comprise a fastening system used to join a front waist region of the reusable outer cover to a back waist region. The fastening system may comprise snaps, buttons, and/or hooks and loops, for example. The insert may comprise a liquid permeable topsheet, a liquid impermeable backsheet, and an absorbent core positioned at least partially intermediate the topsheet and the backsheet. At least one acquisition and distribution system 38 is provided in between the topsheet and the absorbent core. The insert may comprise one or more pairs of leg cuffs and may be free of ears, side panels, and/or waistbands. In some instances, a nonwoven material may be positioned on a garment-facing side of the backsheet. A garment-facing surface of the insert may be attached to a body-facing surface of the reusable outer cover via adhesives, hook and loop fasteners, or other methods of joinder. An example insert and reusable outer cover system is disclosed in U.S. Patent No. 9,011,402, issued on April 21, 2015, to Roe et al. The insert or the reusable outer cover may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

Belts

Referring to Figs. 7 and 8, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or, may alternatively, run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. In other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in U.S. Pat. Appl. Pub. No. 2013/0211363.

Front and back belt end edges 55 and 57 may extend longitudinally beyond the front and back chassis end edges 19 and 21 (as shown in Fig. 6) or they may be co-terminus. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in U.S. Pat. No. 7,901,393, the longitudinal length (along the central longitudinal centerline 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 7 and a gap between the front and back inner belt layers 66 and 67 is shown in Fig. 8.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Figs. 4 and 5).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt seams 15 and 17; or, alternatively, adjacent to the seams 58, 15, and 17 in the manner described in U.S. Pat. No. 9,498, 389 to create a more underwear-like article. The graphics may also be discontinuous.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Pat. Nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

Top sheet

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet 26 is liquid permeable, permitting liquid bodily exudates to readily penetrate through its thickness. A suitable topsheet 26 may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet 26 may have one or more layers. The topsheet 26 may be apertured (Fig. 2, element 31), may have any suitable three-dimensional features, and/or may have a plurality of embossments (e.g., a bond pattern). The topsheet 26 may be hydrophilic or hydrophobic or may have hydrophilic and/or hydrophobic portions or layers. If the topsheet 26 is hydrophobic, typically apertures will be present so that bodily exudates may pass through the topsheet 26.

The topsheet 26 may have an opacity of at least 15 %, or at least 18 %, or at least 20%, or at least 25% as determined according to the opacity test method set out below.

The topsheet 26 of the absorbent article of the present invention may not comprise lotion.

Backsheet

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

Outer Cover Material

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features. The outer cover material 40 may be a hydroentangled nonwoven material.

Absorbent Core

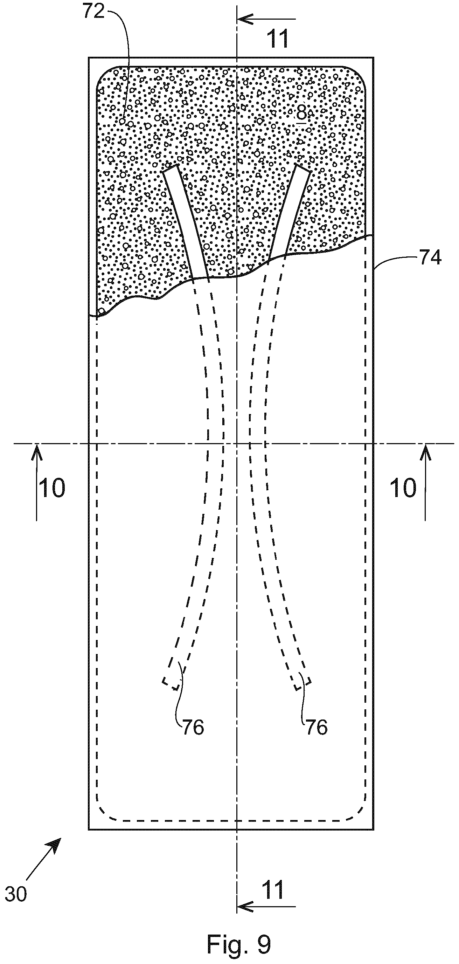

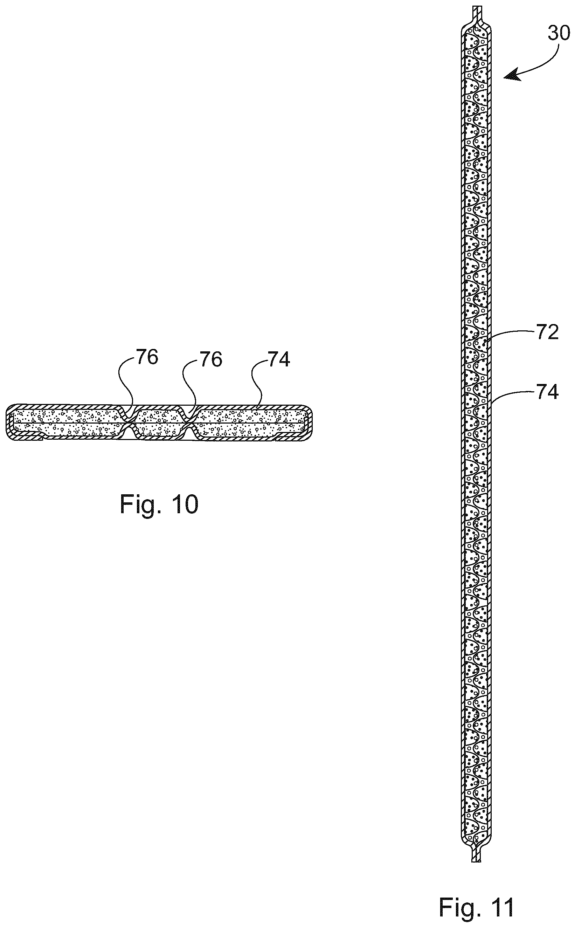

As used herein, the term "absorbent core" 30 refers to the component of the absorbent article 10 intended to store the liquid that enters the absorbent article during use (thus generally having the most absorbent capacity) and that comprises an absorbent material. Referring to Figs. 9-11, in some instances, absorbent material 72 may be positioned within a core bag or a core wrap 74 (the core bag or core wrap being comprised by the absorbent core). The absorbent material may be profiled or not profiled, especially along the longitudinal centerline, depending on the specific absorbent article. "Profiled" means that the absorbent material is not homogeneously distributed across the surface area of the absorbent core. The absorbent core 30 may comprise, consist essentially of, or consist of, a core wrap, absorbent material 72, and glue enclosed within the core wrap. The absorbent material may comprise or consist of a) superabsorbent polymer particles and/or superabsorbent fibers, or b) a mixture of superabsorbent polymer particles and air felt, or c) only air felt, or d) a high internal phase emulsion foam, or e) combinations of any of a) to d). In some instances, the absorbent material may comprise at least 80%, at least 85%, at least 90%, at least 95%, at least 99%, or up to 100% superabsorbent polymers, by weight of the absorbent material. In such instances, the absorbent material may be free of air felt, or at least mostly free of air felt. The absorbent core periphery, which may be the periphery of the core wrap, may define any suitable shape, such as rectangular "T," "Y," "hour-glass," or "dog-bone" shaped, for example. Preferably, the absorbent core has a rectangular shape. An absorbent core periphery having a generally "dog bone" or "hour-glass" shape may taper along its width towards the crotch region 14 of the absorbent article 10.

Referring to Figs. 9-11, the absorbent core 30 may have areas with reduced caliper (wherein the term "reduced" includes areas with no caliper, i.e. areas free of the material of the absorbent core). Areas with reduced caliper may be areas having little or no absorbent material 72, where a body-facing surface of the core bag 74 may be joined to a garment-facing surface of the core bag 74. These areas having little or no absorbent material may be referred to as "channels" 76. These channels can embody any suitable shapes and any suitable number of channels may be provided. In other instances, though less preferred, the absorbent core may be embossed to create the impression of channels. The absorbent core in Figs. 9-11 is merely an example absorbent core. Many other absorbent cores with or without channels are also within the scope of the present disclosure.

Barrier Leg Cuffs/Leg Elastics

Referring to Figs. 1 and 2, for example, the absorbent article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the absorbent article 10 so it can extend upwards from a body-facing surface 4 of the absorbent article 10 and provide improved containment of body exudates approximately at the junction of the torso and legs of the wearer. The barrier leg cuffs 32 are delimited by a proximal edge joined directly or indirectly to the topsheet 26 and/or the backsheet and a free terminal edge, which is intended to contact and form a seal with the wearer's skin. The barrier leg cuffs 32 may extend at least partially between the front waist edge 18 and the back waist edge 20 of the absorbent article 10 on opposite sides of the central longitudinal centerline 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front waist edge 18 and the back waist edge 20. The leg elastics 34 essentially cause portions of the absorbent article 10 proximate to the chassis side edges 22, 24 to help form a seal around the legs of the wearer. The leg elastics 34 may extend at least within the crotch region 14.

Elastic Waistband

Referring to Figs. 1 and 2, the absorbent article 10 may comprise one or more elastic waistbands 36. The elastic waistbands 36 may be positioned on the garment-facing surface 2 or the body-facing surface 4. As an example, a first elastic waistband 36 may be present in the front waist region 12 near the front belt waist edge 18 and a second elastic waistband 36 may be present in the back waist region 16 near the back waist edge 20. The elastic waistbands 36 may aid in sealing the absorbent article 10 around a waist of a wearer and at least inhibiting bodily exudates from escaping the absorbent article 10 through the waist opening circumference. In some instances, an elastic waistband may fully surround the waist opening circumference of an absorbent article.

Acquisition and distribution system

Referring to Figs. 1, 2, 7, and 8, at least one acquisition and distribution system 38 is provided between the topsheet 26 and the absorbent core 30 and may be present at least partially intermediate the topsheet 26 and the absorbent core 30. The acquisition and distribution system comprises acquisition and/or distribution materials which are typically hydrophilic materials that provide significant wicking of bodily exudates. These materials may dewater the topsheet 26 and quickly move bodily exudates into the absorbent core 30. The acquisition and/or distribution materials of the acquisition and distribution system may comprise one or more nonwoven materials, foams, cellulosic materials, cross-linked cellulosic materials, air laid cellulosic nonwoven materials, spunlace materials, or combinations thereof, for example. Typically, the one or more layers of the acquisition and distribution system may each have a width and length that are smaller than the width and length of the topsheet 26. The acquisition and distribution system may have one or more areas with reduced caliper (wherein the term "reduced" includes areas with no caliper, i.e. areas free of the material of one, more than one, or all layers of the acquisition and distribution system), such as channels, as described above with reference to the absorbent core 30 (including the embossed version). The channels in the acquisition and distribution system may align or not align with channels in the absorbent core 30. In an example, a first layer of the acquisition and distribution system 38 may comprise a nonwoven material and a second layer of the acquisition and distribution system may comprise a cross-linked cellulosic material.
The second layer of the acquisition and distribution system may be provided between the first layer of the acquisition and distribution system and the absorbent core. The first layer of the acquisition and distribution system may be provided between the topsheet and the second layer of the acquisition and distribution system.

Landing Zone

Referring to Figs. 1 and 2, the absorbent article 10 may have a landing zone area 44 that is formed in a portion of the garment-facing surface 2 of the outer cover material 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover material 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or vice versa.

Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

Front and Back Ears

Referring to Figs. 1 and 2, as referenced above, the absorbent article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. The ears, or portions thereof, may be elastic or may have elastic panels. In an example, an elastic film or elastic strands may be positioned intermediate a first nonwoven material and a second nonwoven material. The elastic film may or may not be apertured. The ears may be shaped. The ears may be integral (e.g., extension of the outer cover material 40, the backsheet 28, and/or the topsheet 26) or may be discrete components attached to a chassis 52 of the absorbent article on a body-facing surface 4, on the garment-facing surface 2, or intermediate the two surfaces 4, 2.

Masking Layer

One or more masking layers or materials may be provided in the absorbent articles 10. A masking layer may be a layer that provides a cushiony feel when the absorbent article is touched from the garment-facing surface 2 or the body-facing surface 4. The masking layer may "mask" a grainy feel potentially caused by the absorbent material 72, such as superabsorbent polymers particles. The masking layer may "mask" bodily exudates from being visible when viewing the body-facing surface 4 or the garment-facing surface 2 of the absorbent article 10. The masking layer may have a basis weight in the range of about 15 g/m$^2$ to about 50 g/m$^2$ or about 15 g/m$^2$ to about 40 g/m$^2$. The masking layer may comprise one or more nonwoven materials (e.g., a hydroentangled nonwoven material), foams, pulp layers, and/or other suitable materials. The masking layer may be the outer cover material 40. The masking layer may be the layer forming the garment-facing side or the body-facing side of the core bag 74. The masking layer may be a separate material positioned intermediate the garment-facing side of the core bag 75 and the liquid impermeable backsheet 28.

Packages

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Arrays

"Array" means a display of packages comprising disposable absorbent articles of different article constructions (e.g., different elastomeric materials [compositionally and/or structurally] in the side panels, side flaps and/or belts flaps, different graphic elements, different product structures, fasteners or lack thereof). The packages may have the same brand and/or sub-brand and/or the same trademark registration and/or having been manufactured by or for a common manufacturer and the packages may be available at a common point of sale (e.g. oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.
Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

Sanitary Napkin

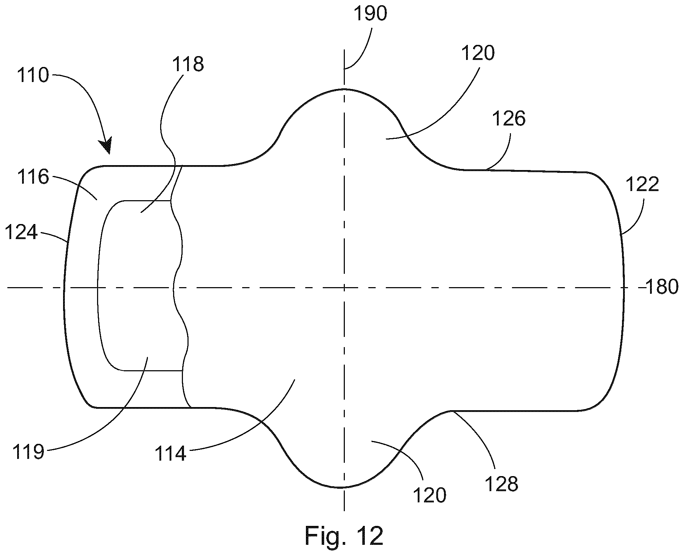

Referring to Fig. 12, an absorbent article of the present disclosure may be a sanitary napkin 110. The sanitary napkin 110 may comprise a liquid permeable topsheet 114, a liquid impermeable, or substantially liquid impermeable, backsheet 116, and an absorbent core 118. The liquid impermeable backsheet 116 may or may not be vapor permeable. The absorbent core 118 may have any or all of the features described herein with respect to the absorbent core 30 and, in some forms, may have a secondary topsheet 119 (STS. The STS 119 may comprise one or more channels, as described above (including the embossed version). In some forms, channels in the STS 119 may be aligned with channels in the absorbent core 118. The sanitary napkin 110 may also comprise wings 120 extending outwardly with respect to a longitudinal centerline 180 of the sanitary napkin 110. The sanitary napkin 110 may also comprise a transverse centerline 190. The wings 120 may be joined to the topsheet 114, the backsheet 116, and/or the absorbent core 118. The sanitary napkin 110 may also comprise a front edge 122, a back edge 124 longitudinally opposing the front edge 122, a first side edge 126, and a second side edge 128 longitudinally opposing the first side edge 126. The longitudinal centerline 180 may extend from a midpoint of the front edge 122 to a midpoint of the back edge 124. The transverse centerline 190 may extend from a midpoint of the first side edge 128 to a midpoint of the second side edge 128. The sanitary napkin 110 may also be provided with additional features commonly found in sanitary napkins as is known in the art.

Examples Cross-sections of Absorbent Articles

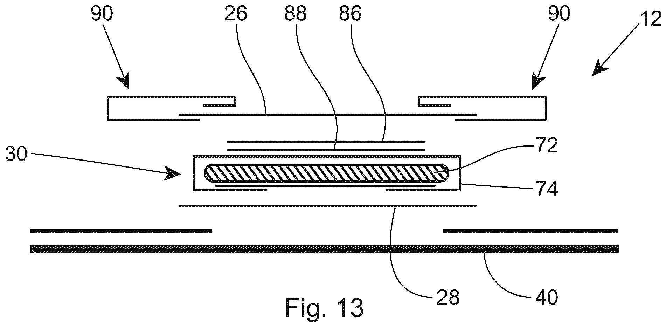

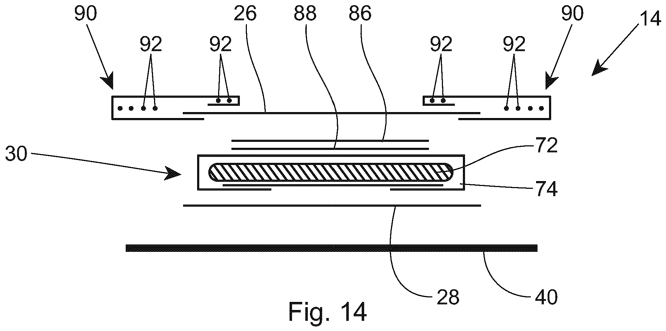

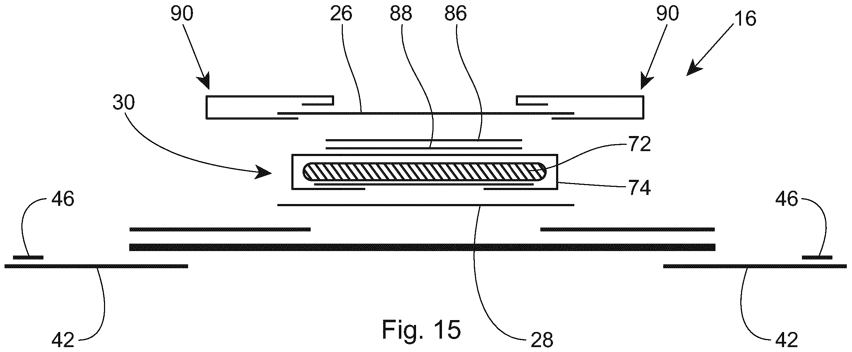

Figs. 13-15 illustrate example cross-sectional views of absorbent articles within the scope of the present disclosure. Fig. 13 is an example cross-sectional view taken within a front waist region 12 of an absorbent article. Fig. 14 is an example cross-sectional view taken within a crotch region 14 of an absorbent article. Fig. 15 is an example cross-sectional view taken within a back waist region 16 of an absorbent article. In Figs. 13-15, an outer cover material is element 40, a topsheet is element 26, a backsheet is element 28, an absorbent core is element 30, with the core bag being element 74, an absorbent material is element 72, and the acquisition and distribution system is element 86. The acquisition and distribution system 86 may comprise cross-linked cellulosic material. An acquisition material is element 88. Barrier leg cuffs are elements 90. Elastics in the barrier leg cuffs are elements 92. Back ears are elements 42. Fasteners on the back ears 42 are elements 46. Construction glues and/or bonds between the various layers and/or components have been removed for clarity. Other cross-sectional configurations known to those of skill in the art are also within the scope of the present disclosure.

Bio-Based Content for Components

Components of the absorbent articles described herein may at least partially be comprised of bio-based content as described in U.S. Pat. Appl. No. 2007/0219521A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and 2016/0206774, and U.S. Pat. No. 9,169,366. Example bio-based polyolefins for use in the present disclosure comprise polymers available under the designations SHA7260TM, SHE150TM, or SGM9450FTM (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

Recycle Friendly and Bio-Based Absorbent Articles

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in U.S. Pat. Appl. Publ. No. 2019/0192723, published on June 27, 2019.

**Test methods**

**Acid Equivalence Test Method, including $pH_0$ and conductivity value determination**

This test method is used to quantify the acid equivalence value after extraction of acquisition and/or distribution layer or of acquisition and/or distribution layer material with deionized water. Furthermore, it includes the measurement of the $pH_0$ value and the measurement of the conductivity value of the filtered liquid part of the extraction mixture.

Specifically, the Acid Equivalence Test Method is used to quantify the presence of acid equivalent in the acquisition and/or distribution system layer or layers and/or in the acquisition and/or distribution system layer material or materials of an absorbent article of interest. The acquisition and/ or distribution layer or layers or the acquisition and/or distribution system layer material or materials of a sample absorbent article are excised and extracted in deionized water, and the extract is titrated with a sodium hydroxide (NaOH) solution of known concentration to establish the mole equivalent of NaOH per gram of acquisition and/ or distribution layer or layers or the acquisition and/or distribution system layer material or materials required to reach pH = 10 ($\pm$0.01) as measured via a suitable pH meter. Furthermore, it is used to quantify the pH value of the filtered liquid part of the extraction mixture before the titration with sodium hydroxide (NaOH) solution, $pH_0$, and to quantify the conductivity value of the extraction mixture before the titration with sodium hydroxide (NaOH) solution.

These tests are each performed under standard lab conditions at 23°C $\pm$2°C, atmospheric pressure, and 45% $\pm$ 10% relative humidity.

Sample Preparation Procedure:

1) The base sample:

For each absorbent article of interest, the acquisition and/ or distribution layer or layers or the acquisition and/or distribution system layer material or materials are excised from one or more sample absorbent articles of interest, in an amount of about 1 gram per extraction with deionized water for the determination of the acid equivalence value.

Generally, this entails removal and discard of the topsheet, the complete absorbent core, the backsheet, layers/ components located between the backsheet and the absorbent core, further components located to the garment-facing / outside-facing side of the backsheet and any layer adhered or glued to the absorbent core. It is generally possible to use ice spray, e.g., non-flammable quick coolant IT ICER Green (PRF), to separate the layers to ease the separation or removal of absorbent article components. Hence, all layers or absorbent article parts not forming part of the acquisition and/or distribution system are removed. Generally, not less than 100 mg of the acquisition and/ or distribution layer or the acquisition and/or distribution system layer material of interest are taken per sample absorbent article of interest.

If the acquisition and/ or distribution layer or the acquisition and/or distribution system layer material of interest can be excised in higher amounts than about 1 gram per sample absorbent article of interest, this higher amount of layer or material is excised and divided into portions of about 1 gram. Generally, amounts of not less than 100 mg, but lower than about 1 gram of acquisition and/ or distribution layer or the acquisition and/or distribution system layer material of interest can be combined to reach amounts of about 1 gram.

Then, all visible particles of superabsorbent polymer are carefully removed from each about 1 gram portion of acquisition and/ or distribution layer or the acquisition and/or distribution system layer material of interest. Layer-like structures or materials, e.g., nonwovens, are cut into pieces having a surface area of about 1 to about 2 $cm^2$ each. Structures that are easily disintegrated, e.g., airlaid layers, are carefully manually separated or lofted up to ensure proper mixing during extraction. The such prepared about 1 gram portions of acquisition and/ or distribution layer or the acquisition and/or distribution system layer material of interest are the base samples.

2) The measurement sample:

1.00 gram ($\pm$ 0.05 gram) of the base sample (as prepared above) is taken and the weight is recorded to the nearest 0.001 gram as ***m_sample.*** This is the measurement sample.

3) The extraction in deionized water:

For each layer or material of interest, 2 measurement samples are taken, s1 and s2.

Each measurement sample is put into a 250 ml Erlenmeyer flask or any suitable container and 200 ml ($\pm$ 0.5 ml) of deionized water are added, e.g., deionized water with purity grade 2 with typical pH in the range of 6.0 to 6.4, e.g., prepared via a lab water purification system (e.g., Milli-Q® from Millipore Corporation). The mixture is stirred for 10 min, e.g., via a suitable magnetic stir bar (e.g., double cross-head stir bar). Each extraction mixture is filtered to remove insoluble components, e.g., using fluted filter paper (e.g., folded qualitative filter paper, type 310, from VWR), so the filtered liquid part of the extraction mixture is obtained (one for measurement sample s1, one for measurement sample s2).

4) The measurement of $pH_0$ and of Conductivity

About 60 to 80 ml of the filtered liquid part of the extraction mixture are filled into a beaker or suitable container of suitable size.

The pH of the filtered liquid part of the extraction mixture is measured via a suitable, calibrated pH meter, e.g., using pH electrode InLab Routine Pro-ISM from Mettler Toledo, and recorded to an accuracy of 0.01. The average (arithmetic mean) of the two pH values (one from s1 extraction, one from s2 extraction) is calculated to an accuracy of 0.1 and recorded as $pH_0$.

The conductivity of the filtered liquid part of the extraction mixture is measured via a suitable conductometer, e.g., using InLab 731-ISM from Mettler Toledo, and recorded to an accuracy of 0.01 mS/cm. The average (arithmetic mean) of the two conductivity values (one from s1 extraction, one from s2 extraction) is calculated to an accuracy of 0.01 mS/cm and recorded as Conductivity.

5) The titration:

First, the required amount of NaOH to reach pH = 10 ($\pm 0.01$) for deionized water is determined. 50 ml ($\pm$ 0.1 ml) of deionized water is titrated - while stirring with a magnetic stir bar of suitable size - utilizing an aqueous sodium hydroxide (NaOH) solution with a concentration of 0.1 mol/l ($\pm$ 0.0005 mol/l), e.g., sodium hydroxide solution c(NaOH)=0.1 mol/l Titripur® from Merck. The NaOH solution is added via a suitable lab pipette, e.g., via an electronic dispenser pipette, e.g., Multipette® Xstream from Eppendorf, using titration mode and Combitips® advanced, size 0.5mL, from Eppendorf, and the pH is measured via a suitable, calibrated pH meter, e.g., using pH electrode InLab Routine Pro-ISM from Mettler Toledo.

The volume of NaOH solution added where a pH value of pH = 10 ($\pm 0.01$) is reached is determined to an accuracy of 0.0005 ml and recorded. The titration of deionized water is repeated for three more times.

The average (arithmetic mean) of four required NaOH solution volumes is calculated to an accuracy of 0.0005 ml and recorded as *V(NaOH)_50ml_blank.*

Second, 50 ml ($\pm$ 0.1 ml) of the filtered liquid part of the extraction mixture is transferred to a separate beaker or suitable container, e.g., a 100 ml titration beaker. The solution is titrated as described above. The volume of NaOH solution added where a pH value of pH = 10 ($\pm 0.01$) is reached is determined and recorded to an accuracy of 0.0005 ml as *V(NaOH)_50ml.* The volume of NaOH solution needed in the titration of deionized water *V(NaOH)_50ml_blank* is subtracted. The remaining volume of NaOH solution *V(NaOH)_50ml_sample* with *V(NaOH)_50ml_sample* = *V(NaOH)_50ml* - *V(NaOH)_50ml_blank*
is used to calculate the molar amount of applied NaOH to the nearest 0.0001 mmol and recorded as *n(NaOH)_50ml.*

The titration of 50 ml ($\pm$ 0.1 ml) of the filtered liquid part of the extraction mixture is repeated.

The Acid Equivalence value

The Individual Acid Equivalence value *IAE* is calculated by multiplying the molar amount of applied NaOH *n(NaOH)_50ml* with 4 to get *n(NaOH)_200ml* and then divided by the mass of the measurement sample *m_sample.* The IAE is calculated to the nearest 0.001 mmol/g and recorded.

As described above, the titration on 50-ml portion of the extraction solution is done twice for both extractions (sample s1 and s2). In total, four (4) IAE values are generated per layer or material of interest.

The average (arithmetic mean) of the four IAE is calculated to the nearest 0.001 mmol/g and recorded as *Acid Equivalence value* of the layer (or material, respectively) of interest.

**Surface pH Test Method**

This test method is used to quantify the surface pH value of an acquisition and/or distribution layer, more specifically the surface pH value of the body-facing surface (pH_bfs) and the surface pH value of the garment-facing surface (pH_gfs).

This test is performed under standard lab conditions at 23°C $\pm$2°C, atmospheric pressure, and 45% $\pm$ 10% relative humidity.

The acquisition and/or distribution layer(s) of interest are excised from the absorbent article as described above in the Acid Equivalence test method.

The surface pH of the layer is measured using a skin and scalp pH meter (catalog number HI981037, Hanna Instruments, Smithfield, RI, USA, or equivalent) on the body-facing surface (pH_bfs) and on the garment-facing surface (pH_gfs).

In case the surface of the layer is too dry for the pH measurement, one droplet of deionized water is placed onto the surface. After a waiting time of three minutes, the pH is measured at the location where the droplet was placed.

The surface pH measurement is repeated three times per surface.

The average (arithmetic mean) of the four surface pH of each surface is calculated to the nearest 0.1 and recorded as surface pH, as pH_bfs for the body-facing surface , and as pH_gfs for the garment-facing surface, respectively, of the layer of interest.

**Centrifuge Retention Capacity (CRC) test method for SAP particles**

Centrifuge Retention Capacity (CRC) test method as set out in EDANA NWSP 241.0.R2(19).

**Superabsorbent Fiber Centrifuge Retention Capacity (SAF-CRC) test method**

Capacity of the superabsorbent fibers, the capacity of the nonwoven web(s) with superabsorbent fibers and the capacity of the absorbent core is determined according to the Centrifuge Retention Capacity (CRC) test method as set out in EDANA NWSP 241.0.R2(19). In deviation from EDANA NWSP 241.0.R2(19) the sampling (chapter 8 in EDANA NWSP 241.0.R2(19) ) for the superabsorbent fibers, nonwoven web(s) with superabsorbent fibers and/or the absorbent core is as following:

The superabsorbent fibers, nonwoven web(s) with superabsorbent fibers and/or the absorbent core are cut into pieces with approximately 5 mm as largest dimension. The cutting can e.g. be done manually with scissors. Care is taken that the fibrous structure (the core, the nonwoven or the bulk of fibers) is not majorly compressed before or during the cutting process. This ensures sufficient void space between the superabsorbent fibers, so they can be predominately wetted by the swelling medium at the entire surface area.

Further deviations from or additions to EDANA NWSP 241.0.R2(19) in the procedure (chapter 9.1-9.5 in EDANA NWSP 241.0.R2(19) ) for the superabsorbent fibers, nonwoven web(s) with superabsorbent fibers and/or the absorbent core are as following:

The sample for the measurement is taken carefully, e.g. with a lab pincet, to put it into the teabag. With a lab pincet, the fibers are carefully distributed in the teabag to avoid lumps and fiber lumps, if any, are carefully opened.

When sealing the teabag, care is taken that no material of the superabsorbent fibers, nonwoven web(s) with superabsorbent fibers and/or the absorbent core is in the area of the seal. This ensures a complete and sufficiently strong sealing of the teabag.

All other items of the test method are executed as set out in EDANA NWSP 241.0.R2(19).

**Urease Activity Test Method**

The Urease Activity Test Method measures urease activity in absorbent articles after treatment with a test solution containing urease and synthetic urine containing urea. In this method, like test samples, excised from like absorbent articles of interest, are placed in test containers and treated with the test solution containing urease and synthetic urine containing urea. A layer of collagen is also present in each test container. Each test container is closed and incubated at 37°C for approximately 4 hours. Thereafter, urease activity is characterized by measuring (a) gas-phase ammonia release, (b) pH of the top-most layer of the absorbent article sample (i.e., the body-facing layer of the absorbent article) and (c) pH of collagen.

Sample and Material Preparation

For each absorbent article of interest, test samples are excised from each of six like absorbent articles of interest. From each like absorbent article of interest, the excised test sample is a circular disk 10.0 cm in diameter and centered at a point lying on the transverse centerline that is a distance rearward of the front edge of the absorbent core of the absorbent article that is 48.5% of the entire length of the absorbent core (i.e., in longitudinal dimension). That is, the point at which each test sample is centered is along the transverse centerline and slightly forward of the intersection of the absorbent core's transverse and longitudinal centerlines. The disk is cut or punched through all layers present in the absorbent article at this location, and the test sample subsequently maintains the internal organization of structure of layers as was present in the intact absorbent article.

Cylindrical, screw-top, polypropylene containers, approximately 500 mL in volume and measuring approximately 10.5 cm in diameter and 8 cm in height, are used to house each test sample during and after addition of test solution. The bottom of the cylindrical containers is large enough in diameter such that a test sample can lie flat. In each lid of the test container is drilled a centered hole 8 mm in diameter. This hole is immediately sealed over with tape and is opened only after incubation of the container at 37 °C.

For each screw-top container to be used during analysis, an annulus (made of acrylic or polycarbonate) is fashioned with an outer diameter of 7.5 cm, an inner diameter of 5 cm, and a thickness of 7.5 mm.

Circular collagen disks, 7.0 cm in diameter, to be positioned individually above each test sample, are cut from commercially available collagen sheets (Naturin COFFI clear, having a basis weight of $33.5 \pm 0.4$ g/m$^2$, Viscofan Group, Tajonar, Spain) or equivalent. Each circular collagen disk is placed on top of an annulus. This assembly allows positioning of the collagen disks above each test sample, such that there is no direct contact between the collagen disk and the test sample.

Synthetic Urine is prepared by dissolving 2% w/w urea, 0.9% w/w sodium chloride, 0.11% w/w magnesium sulfate monohydrate, 0.079% w/w calcium chloride monohydrate in deionized water.

Urease stock solution (23.5 U/ml) is made by dissolving Jack Bean Urease (Catalog number U1500, Millipore Sigma, St. Louis, MO, USA, or equivalent) in deionized water.

For each sample to be tested, 75 ml of the test solution is prepared immediately before use by mixing 67.5 ml of Synthetic urine and 7.5 ml of urease stock solution at ambient lab temperature ($23\pm1$ °C).

Test Procedure

Each test sample is placed in the above-mentioned cylindrical screw-top container with its skin-facing side facing upward. 75 mL of test solution (prepared just before use) is then poured over the test sample. Within one minute, after visually confirming that the test solution is fully absorbed by the test sample and no free liquid is present above the test sample, the annulus bearing a single layer of collagen is placed above the test sample such that there is no direct contact between the collagen disk and the test sample. In the event that free test solution remains visible in the test container after one minute, the free test solution is removed using appropriate means such as a pipette and the annulus bearing a layer of collagen placed above the test sample. A container lid is immediately used to close the test container, and the test container is incubated at 37 °C for $4.0 \pm 0.25$ hours in a climate-controlled oven/incubator.

Measurement and Reporting

Immediately following the incubation period, the test container for each test sample being characterized is removed from the oven/incubator and measured.

For each test container and test sample, the concentration of gas-phase ammonia in the headspace in the test container above the test sample is measured using the Draeger flow-tube-based gas-phase ammonia detection system (for example, that available from Draegerwerk AG & Co. KGaA, Luebeck, Germany). Suitable apparatus for this particular application are Draeger short-term detection tubes - Ammonia 5/b, catalog no. 81 01 941 and Draeger Accuro Pump, catalog no. 64 00 000, or equivalent. The tape sealing the orifice in the lid of the test container is removed and the ammonia tube is inserted to a depth of $3\pm0.5$ cm into the test container. The Draeger pump is used to withdraw a sample of the gas-phase in the test container as per the manufacturer's instructions, drawing 100 ml two times (i.e, 200 ml) and then divide the level of color change by 2 to obtain the ppm value for ammonia. Depending on the concentration of headspace ammonia, it may be necessary to use alternative short term ammonia detection tubes, suited to the concentration being measured. The level of colour change in the ammonia tube is used to determine the amount of ammonia (ppm) in the gas-phase of the container. For each test container and test sample, the gas-phase ammonia concentration is recorded to the nearest 1 ppm.

Following the measurement of gas-phase head-space ammonia (approximately 30-60 minutes), for each test container and test sample, the lid of each test container is unscrewed and removed. The collagen disk and annulus are withdrawn/extracted from the test container so as to avoid contact between the collagen to the sides of the container. The pH of the collagen layer is measured using a skin and scalp pH meter (catalog number HI981037, Hanna Instruments, Smithfield, RI, USA, or equivalent). In general, the collagen will have developed some tack during the incubation and adheres to the annulus, thereby supporting modest pressure against the collagen surface. This allows collagen to be supported on the ring when the pH probe is in contact to the collagen disk for pH measurement. The pH meter is calibrated before measurements with buffer solutions of pH 4 and 7 per the instructions of the manufacturer. The pH of the collagen associated with each test sample is recorded to the nearest 0.01 of the test sample collagen pH.

Finally, for each test sample, the pH of the topmost layer of absorbent article test sample is measured using the skin and scalp pH meter. While the pH meter is placed in contact with the topmost layer of the test sample, the entire test sample (all layers) remains present intact underneath the topmost layer. The pH of the topmost layer of each test sample is recorded to the nearest 0.01 of the test sample topsheet pH.

The arithmetic mean of the gas-phase ammonia concentration among all test samples analyzed is calculated and reported to the nearest 0.1 ppm as the gas-phase ammonia concentration of the sample absorbent article. The arithmetic mean of the topsheet pH among all test samples analyzed is calculated and reported to the nearest 0.1 as the topsheet pH of the sample absorbent article.

The arithmetic mean of the collagen pH among all test samples analyzed is calculated and reported to the nearest 0.1 as the collagen pH due to the sample absorbent article.

**Modified Fluid Acquisition Test**

The Modified Fluid Acquisition ("MFA") Test is designed to measure the speed at which 0.9 weight-% saline solution is absorbed into an absorbent core that is compressed at 2.07 kPa. Additional layers may be placed on top and/or below the absorbent core for the Modified Fluid Acquisition Test (see Examples below), in which case the MFA determines the speed at which 0.9 weight-% saline solution is absorbed into an absorbent core and the additional layers that is compressed at 2.07 kPa.

A known volume is introduced four times, each successive dose starting five (5) minutes after the previous dose has absorbed. Times needed to absorb each dose are recorded. The test fluid is 0.9 weight-% w/v saline solution and is prepared by weighing 9.0 g $\pm$ 0.05g of NaCl into a weigh boat, transferring it into a 1L volumetric flask, and diluting to volume with de-ionized water.

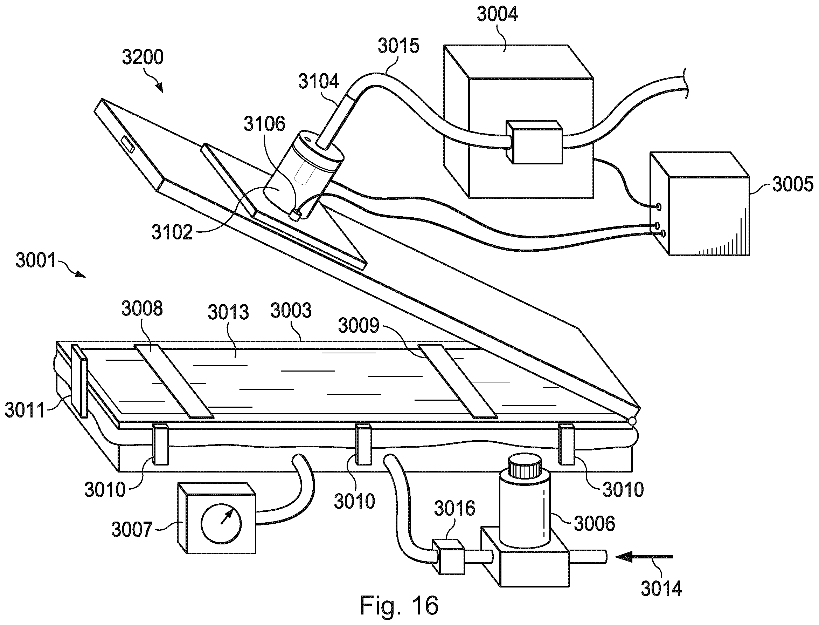

The MFA apparatus is depicted in FIG. 16 through FIG. 18B. The MFA apparatus comprises a bladder assembly 3001 and a top plate assembly 3200 that includes a deposition assembly 3100. A controller 3005 is used to 1) monitor the impedance across electrodes 3106, recording the time interval 0.9 weight-% saline solution is in a cylinder 3102, 2) interface with a liquid pump 3004 to start/stop dispensing, and 3) time intervals between dosing. The controller 3005 is capable of recording time events to $\pm$ 0.01 sec. A house air supply 3014 is connected to a pressure regulator 3006 capable of delivering air at a suitable flow/pressure to maintain 2.07 kPa in the bladder assembly 3001. A liquid pump 3004 (Ismatec MCP-Z gear pump, available from Cole Palmer, Vernon Hills, IL or equivalent) capable of delivering a flow of 10-80 mL at a rate of 3-15 mL/s is attached to a stainless-steel tube 3104 of the deposition assembly 3100 via Tygon® tubing 3015.

The bladder assembly 3001 is constructed of 12.7 mm Plexiglas with an overall dimension of 80 cm long by 30 cm wide by 10 cm tall. A manometer 3007 to measure the pressure inside the assembly and a pressure gauge 3006 to regulate the introduction of air into the assembly are installed through two holes through the light side. A bladder 3013 is assembled by draping a 50 mm by 100 mm piece of silicone film, (thickness 0.02", Shore A durometer value of 20, available as Part# 86435K85 from McMaster-Carr, Cleveland, OH) over the top of the box with enough slack that the film touches the bottom of the box at its center point. An aluminum frame 3003 with a flange is fitted over the top of the film and secured in place using mechanical clamps 3010.

When in place, the assembly should be leak free at a pressure of 3.45 kPa. A front 3008 and back 3009 sample support of 5 cm by 30 cm by 1 mm are used to anchor the sample. The absorbent core (optionally with additional layers on top and/or below) is attached to the top surface of the sample supports by either adhesive tape or mechanical "hook" fasteners. These supports can be adjusted along the length of the aluminum frame 3003 via a simple pin and hole system to accommodate different size absorbent cores and to correctly align their loading point.

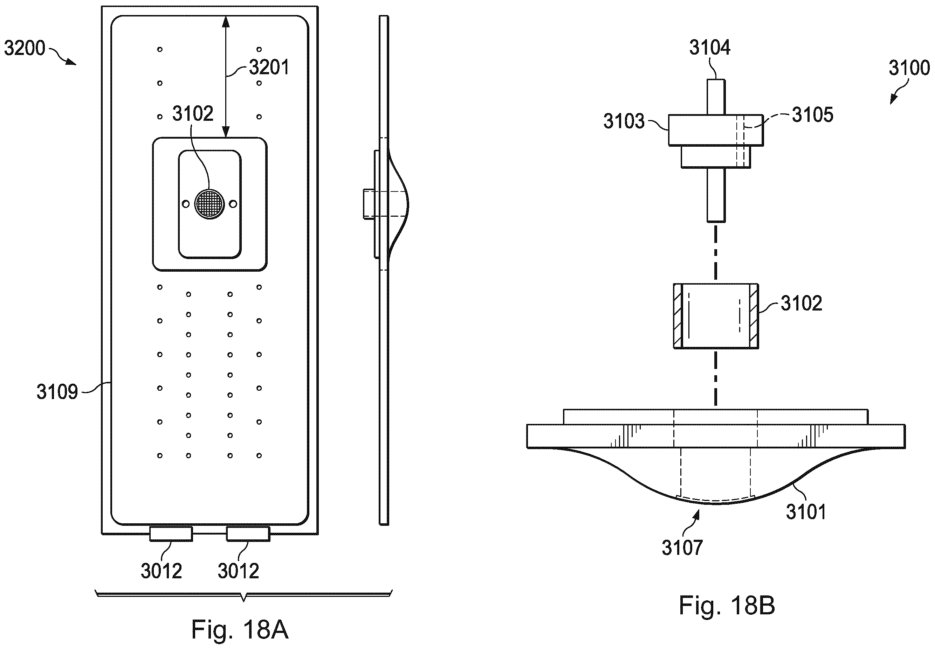

The top plate assembly 3200 is constructed of an 80 cm by 30 cm piece of 12.7 mm Plexiglas reinforced with an aluminum frame 3109 to enhance rigidity. The plate has a cutout l70 mm wide by 201 mm long centered laterally on the plate, l70 mm from the front of the plate 3201 for mounting of the deposition assembly. In addition, the top plate has thirty-six (36) 3.2 mm diameter holes drilled through it distributed as shown in FIG. 18A. The holes prevent air from being trapped under the top plate as the bladder is inflated. The top plate assembly 3200 is connected to the bladder assembly 3001 via two hinges 3012. During use, the top assembly is closed onto the bladder assembly and locked into place using a mechanical clamp 3011.

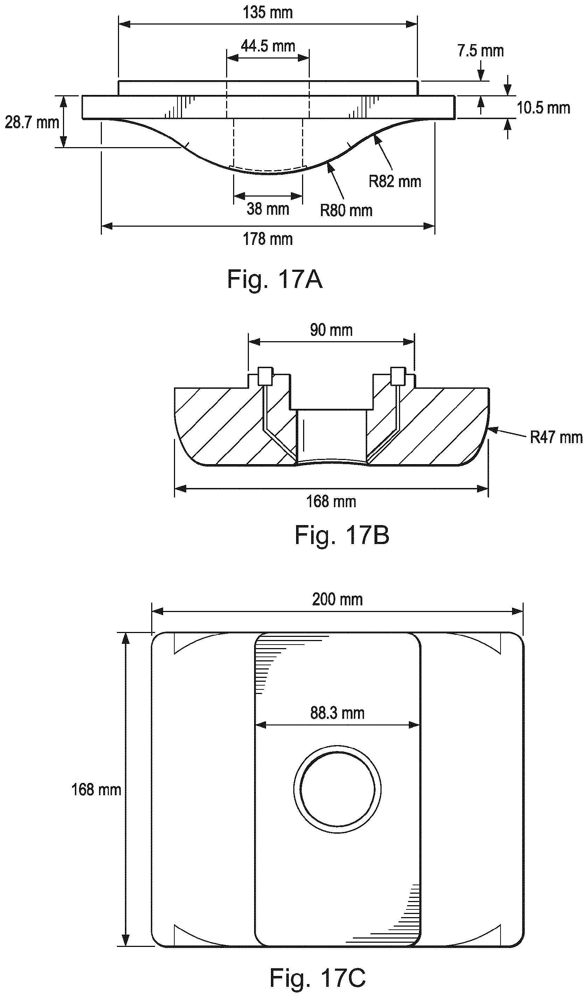

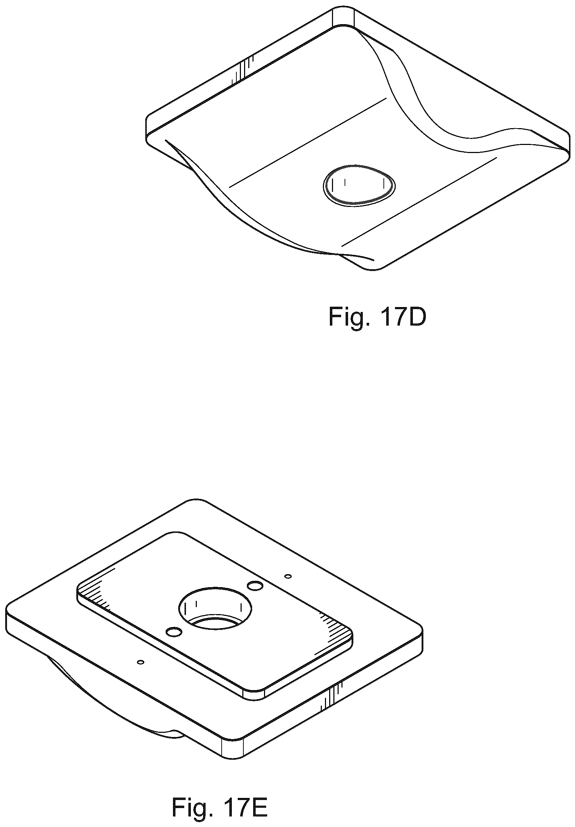

The deposition assembly 3100 is fitted into the top plate 3200 and includes 1) a liquid introduction cylinder 3102, 2) a curved surface 3101 at the loading point of the absorbent core and 3) electrodes 3106 that are used to detect fluid in the cylinder 3102. The detailed dimensions of the curved component are provided in FIG. 17A to FIG. 17E. FIG. 17A is a side view of the curved component. FIG. 17B is an end view of the curved component. FIG. 17C is a bottom view of the curved component. FIG. 17D is a bottom perspective view of the curved component. FIG. 17E is a top perspective view of the curved component. This curved component can be milled or 3D printed. The top portion of the introduction cylinder is a 50.8 mm O.D. Plexiglas cylinder 3102 with a 38.1 mm I.D. This is fitted into the curved component to give the introduction cylinder a total height of 100 mm. Imbedded electrodes run from connectors on the upper surface of the curved component and terminate flush with an inside wall of the introduction cylinder, 2 mm from the bottom of the cylinder. The two electrodes are positioned 180 degrees apart. A nylon screen 3107 is cut and affixed flush with the bottom of the cylinder such that the sample cannot swell into the cylinder. A 5 mm semi-circle is cut in the screen in the immediate area of the two electrodes. The deposition assembly is inserted into the top plate as shown in FIG. 18A such that the curved surface is flush with the bottom of the top-plate assembly 3200. The introduction cylinder 3102 is topped with a loose-fitting nylon cap 3103. The cap has a 6.35 mm O.D. steel tube 3104 inserted through its center. When the cap is in place, the bottom of the tube ends 20 mm above the screen 3107. The cap also has an air hole 3105 to ensure negative pressure does not impede the absorption speed.

Place the absorbent core flat onto a lab bench and identify the intersection of the longitudinal centerline with the loading point as defined in the following Table.

Conditions for Modified Fluid Acquisition Testing:

| Loading Point from front of Core | Gush Volume | Flow Rate | Delivery Time | Number of subsequent gushes |
|---|---|---|---|---|
| mm | mL | mL/s | s | |
| 170 | 75 | 15 | 5 | 4 |

Attach the end of the absorbent core, which is intended to be placed towards the front end of the absorbent article (i.e., the front waist region for a diaper or pant), to the top surface of the front sample plate 3008 by either adhesive tape or mechanical "hook" fasteners with a topsheet facing upward. For absorbent cores which are symmetric along their transverse axis, it is not relevant which end of the absorbent core is attached to the top surface of the front sample plate 3008. The placement is such that no parts of the absorbent core overlay the plate. The sample plate 3008 is attached to the aluminum frame 3003 such that the size-dependent (i.e., size of the absorbent article into which the absorbent core is intended to be provided) Loading Point (as defined in the Table above) of the absorbent core will be centered longitudinally and laterally within the cylinder 3102 when the top plate assembly has been closed. The end of the absorbent core, which is intended to be placed towards the back end of the absorbent article, is secured to the back sample plate 3009 by either adhesive tape or mechanical "hook" fasteners, once again ensuring that no parts of the absorbent core overlay the plate. The back sample plate 3009 is then attached to the aluminum frame 3003 such that the absorbent core is taunt but not stretched. The top plate assembly is closed and fastened, and the bladder is inflated to 2.07 kPa $\pm$ 0.07 kPa. The pressure is maintained at this level during the complete loading sequence of the test.

The pump 3004 is primed and then calibrated to deliver the size-dependent volume and flow rate selected from the Table above. Volume and flow rate must be within $\pm$ 2% of target. The cap 3103 is placed into the cylinder 3102. The controller 3005 is started, which in turn delivers the first dose of 0.9 weight-% saline solution. After the volume has been absorbed, the controller waits for 5.0 minutes before addition of the next dose. This cycle is repeated for a total of four doses. If the fluid leaks out of or around the article (i.e., is not absorbed into the article) then the test is aborted. Also, if any acquisition time exceeds 1200 seconds, the test is aborted. The acquisition time is defined as the difference between the start time (i.e., when the 0.9 weight-% saline is first introduced into the cylinder and that conducting fluid completes the circuit between the electrodes) and the stop time (i.e., when the fluid has completely drained from the cylinder and the circuit between the electrodes is broken). Acquisition times are recorded by the controller for each dose to the nearest 1.0 second. After the last dose is acquired, pressure is applied for an additional 10 minutes. Open the pressure relief valve 3016 to deflate the bladder and then remove the sample from the acquisition system.

In the same fashion, run a total of four (4) replicates for each absorbent core to be evaluated. Calculate and report the Acquisition Times (sec) for each dose as the arithmetic mean of the replicates to the nearest 1.0 sec.

As said above, additional layers may be placed on top of the absorbent core, prior to testing, such as topsheet materials and/or layers that are used as acquisition materials in an absorbent article. Also, a liquid impervious polyolefin film may be provided underneath the absorbent core, i.e., between the absorbent core and the sample plate.

**Post Acquisition Collagen Rewet Test Method**

This method requires a collagen film having a Fixed Height Frit Absorption (FHFA at 0cm) between 0.48 g/g and 0.66 g/g and FHFA at 20cm between 0.15g/g and 0.21 g/g as measured according to the method described below. The collagen film has also a basis weight of 31.5 +/-3.5 g/m$^2$. The collagen film can be purchased from Viscofan Group, 31192 Tajonar-Navarra, Spain, under the designation of Naturin COFFI clear, or equivalent material having the characteristics and basis weight as described above.

Before executing the test, the collagen film as is prepared by being cut into circular sheets of 90 mm (3.54 inches) diameter e.g. by using a sample cutter device, and by equilibrating the film in the controlled environment of the test room (see Modified Fluid Acquisition Test Method) for at least 12 hours (tweezers are to be used for all handling of the collagen film).

At least 5 minutes, but not more than 6 minutes after the last gush, which has been performed in the above Modified Fluid Acquisition Test Method, is absorbed, the cover plate and weights are removed, and the test sample is carefully placed flat on a lab bench.

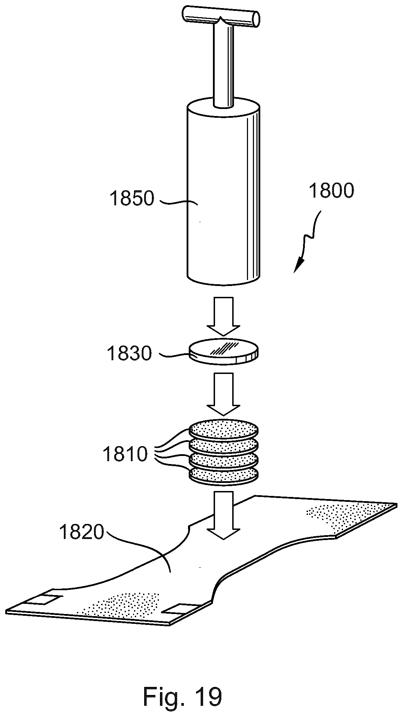

Four sheets of the precut and equilibrated collagen material 1810 are weighed with at least one milligram accuracy, and then positioned centered onto the loading point of the article, as defined in the Modified Fluid Acquisition Test Method, and covered by a plate 1830 made of Poly(methyl methacrylate) (PMMA) (e.g. Perspex®) of 90 mm (3.54 inches) diameter, and about 20 mm (0.78 inches) thickness. A weight (1850) of 15 kg is carefully added (also centered). After 30 +/-2 seconds the weight and Perspex® plate are carefully removed again, and the collagen films are reweighed (See the system 1800 in Figure 19).

The Rewet result is the moisture pick up of the collagen film, expressed in mg. Four products for each option are tested in this fashion and the average rewet is calculated.

**Fixed Height Frit Absorption (FHFA) at 20 cm and at 0 cm Test Methods**

This test is suitable of measuring the uptake of a material under the conditions of suction pressures of 20 cm or of 0 cm of fluid, for example of a saline solution (0.9% wt. NaCl solution) after 30s.

*General apparatus setup*

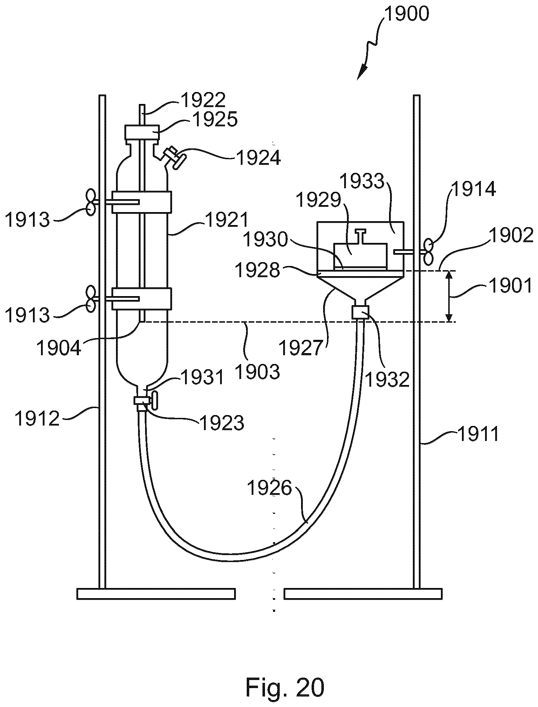

Figure 20 shows the FHFA measurements setup 1900: a suitable fluid delivery reservoir 1921, has an airtight stopcock 1924 to allow the air release during the filling of the equipment. An open-ended glass tube 1922 having an inner diameter of 10 mm extends through a port 1925 in the top of the reservoir such that there is an airtight seal between the outside of the tube and the reservoir, this allows maintaining the required zero level of the hydro head during the experiment regardless the amount of liquid in the reservoir. Reservoir 1921 is provided with delivery tube 1931 having an inlet at the bottom of the reservoir, a stopcock 1923, with the outlet connected to the bottom 1932 of the sample holder funnel 1927 via flexible plastic tubing 1926 (e.g., Tygon®). The Fluid reservoir is firmly held in position by means of standard lab clamps 1913 and a suitable lab support 1912. The internal diameter of the delivery tube 1931, stopcock 1923, and flexible plastic tubing 1926 enables fluid delivery to the sample holder funnel 1927 at a high enough flow rate such that such flowrate is higher than the flowrate absorbed by the collagen sample in the conditions of the experiment and exclude that the measured uptake is limited by the fluid flowrate supplied by the equipment system. The reservoir 1921 has a capacity of approximately 1 liter. Other fluid delivery systems may be employed provided that they are able to deliver the fluid to the sample holder funnel 1927 maintaining the zero level of the hydrostatic liquid pressure 1903 at a constant height during the whole experiment.

The sample holder funnel 1927 has a bottom connector with an internal diameter of 10 mm, a measurement and a chamber 1933 where a glass frit 1928 is accommodated. The sample holder chamber has a suitable size to accommodate the sample 1930 and the confining pressure weight 1929. The frit is sealed to the wall of the chamber 1933. The glass frit has pore of specific size of 16-40μm (glass frit type P 40, as defined by ISO 4793) and a thickness of 7 mm.

The confining pressure weight 1929 is a cylinder with a diameter identical to the sample size (6 cm) and a weight of 593.94 g so to apply exactly 2.06 kPa of confining pressure to the sample 1930. The sample holder funnel 1927 is precisely held in position using a suitable lab support 1911 through a standard lab clamp 1914. The clamp should allow an easy vertical positioning of the sample holder funnel 1927 such that the top of the glass frit 1928 can be positioned at a) the same height (+/- 1 mm) of the bottom end 1904 of the open-ended glass tube 1922 and b) exactly 20 cm (+/- 1 mm) above the bottom end 404 of the open-ended glass tube 1922. Alternatively, two separated clamps are positioned at the abovementioned setups a and b and the sample holder funnel is alternatively moved from one to the other. During the non-usage time, the instrument is kept in proper operating conditions flooding the sample holder funnel 1927 with an excess of liquid to guarantee a proper wetting of the glass frit 1928 that should be completely below the liquid level. The sample holder funnel 1927 is also covered with an airtight cap (not shown) to avoid evaporation and therefore a change in solution salinity. During storage stopcocks 1923 and 1924 are also accordingly closed to avoid evaporation as well as the open-ended tube 1922 airtight sealed with a cap (not shown).

*Sample preparation*

During the sample preparation, the sample is only touched with the tweezers. Discs of 6 cm diameter are cut out of the collagen material using any suitable die cutter. The samples are then stored in a closed container, e.g., a petri dish with lid, and conditioned in the controlled environment of the test room for at least 24 hours.

*Material used:*

Saline solution at a concentration of 0.9% by weight

FHFA equipment (as set out above)
Bubble level
Analytical balance with a resolution of $\pm$ 0.001 g with air draft protections.
Funnel

Tweezers
Timer

*Experiment Setup*

Before starting the experiment:

1) The caps to the open-ended tube 1922 and the sample holder funnel 1927 are removed.

2) Ensuring the stopcock 1923 is closed, the stopcock 1924 is opened to allow the air to flow out of the liquid reservoir as displaced by liquid during the refilling phase. The liquid reservoir 1921 is refilled through top end of the open-end tube 1922 with the 0.9 % Saline solution with the help of suitable means such a funnel (not shown) at the end of the filling the stopcock 1924 is closed.

If during all the experiments the liquid level would be close to the bottom 1904 of the open-ended tube 1922, before running the next sample, the liquid reservoir must be refilled repeating this step number 2.

3) The sample holder funnel 1927 is removed from the lab clamp 1914 and the excess of liquid is removed pouring it away.

4) Manually holding the sample holder funnel 1927 such that the top of the glass frit 1928 lies around 20 cm below the bottom end 1904 of the open-ended tube 1922 the stop cock 1923 is carefully open until the air liquid interface in the open-ended tube 1922 reaches the bottom end 1904 and a few bubbles of air escape from tube 1922. At this point the stop cock 1923 is closed.

5) The excess of liquid now present in the sample holder funnel 1927 is again disposed and the system is now ready to start the measurements.

For measuring the Fixed Height Frit Absorption (FHFA) at 20 cm, for each replicate:

1) The sample holder is positioned on the clamp 414 such that the top of the glass frit 1928 lies exactly 20 cm (+/- 1 mm) above the bottom end 404 of the open-ended tube 1922. To ensure a reliable measure it is checked that the glass frit 1928 is perfectly horizontal with the help of a bubble level.

2) Any remaining droplets of liquid on top of the glass frit are carefully removed by means of a filter paper of any other suitable material.

3) The sample is weighed with an analytical balance with a resolution of $\pm$ 0.001 g. The Weight is recorded as Dry Sample Weight ($W_D$) to the nearest 0.001 g when the readings on the balance become constant.

4) 4 sheets of collagen material are carefully aligned on top of each other using tweezers. This stack of 4 sheets of collagen is subsequently referred to as "sample". The sample 1930 is positioned in the center of the sample holder with the help of tweezers with particular care in not altering the orientation and relative position of each of the layers of the acquisition system.

5) The confining weight 1929 is positioned centered on the sample

6) The stopcock 1923 is opened for 30 +/- 1 seconds allowing liquid to flow in the sample and then closed again.

7) The confining weight 1929 and the sample 1930 are carefully removed from the glass frit 1928 with the help of tweezers.

8) The sample 1930 is weighed with the analytical balance with a resolution of $\pm$ 0.001 g. The Weight is recorded as 20 cm Sample Weight ($W_{20}$) to the nearest 0.001 g when the readings on the balance become constant.

The measurements of a sample are now completed, and a subsequent replicate can be measured repeating the above steps. Once terminated the series of experiment around 1 cm of liquid is added on the Sample Holder funnel 1927 to completely submerge the glass frit 1928. All the stopcocks are closed and the cap positioned according to the storage condition explained above to avoid evaporation and ensure reliability of the subsequent measurements.

*Calculations:*

The FHFA at 20 cm ($FHFA_{20}$) is defined according to the following formula: $FHFA_{20} = (W_{20} - W_D)/W_D$ and has unit of g/g.

For measuring the Fixed Height Frit Absorption (FHFA) at 0 cm, for each replicate:

1) The sample holder is positioned on the clamp 1914 such that the top of the glass frit 1928 lies exactly 0 cm (+/- 1 mm) above the bottom end 404 of the open-ended tube 1922. To ensure a reliable measure it is checked that the glass frit 1928 is perfectly horizontal with the help of a bubble level.

2) Any remaining droplet of liquid on top of the glass frit are carefully removed by means of a filter paper of any other suitable material.

3) The sample is weighed with an analytical balance with a resolution of $\pm$ 0.001 g. The Weight is recorded as Dry Sample Weight ($W_D$) to the nearest 0.001 g when the readings on the balance become constant.

4) 4 sheets of collagen material are carefully aligned on top of each other using tweezers. This stack of 4 sheets of collagen is subsequently referred to as "sample". The sample 1930 is positioned in the center of the sample holder with the help of tweezers with particular care in not altering the orientation and relative position of each of the layers of the acquisition system. It is important that the topsheet facing side of each layer is facing now downwards during the experiment in the direction of the glass frit 1928, reproducing the liquid flow entrance direction correctly.

5) The confining weight 1929 is positioned centered on the sample

6) The stopcock 1923 is opened for 30 +/- 1 seconds allowing liquid to flow in the sample and then closed again.

7) The confining weight 1929 and the sample 1930 are carefully removed from the glass frit 1928 with the help of tweezers.

8) The sample 1930 is weighed with the analytical balance with a resolution of $\pm$ 0.001 g. The Weight is recorded as 0 cm Sample Weight ($W_0$) to the nearest 0.001 g when the readings on the balance become constant.

The measurements of a sample are now completed, and a subsequent replicate can be measured repeating the above steps. Once terminated the series of experiment around 1 cm of liquid is added on the Sample Holder funnel 1927 to completely submerge the glass frit 1928. All the stopcocks are closed, and the cap positioned according to the storage condition explained above to avoid evaporation and ensure reliability of the subsequent measurements.

*Calculations:*

The FHFA at 0 cm ($FHFA_0$) is defined according to the following formula: $FHFA_0 = (W_0 - W_D)/W_D$ and has unit of g/g.

**IPRP Test Method**

Permeability generally refers to the quality of a porous material that causes it to allow liquids or gases to pass through it and, as such, is generally determined from the mass flow rate of a given fluid through it. The permeability of an absorbent structure is related to the material's ability to quickly acquire and transport a liquid within the structure, both of which are key features of an absorbent article. Accordingly, measuring permeability is one metric by which a material's suitability for use in absorbent articles may be assessed.

The following test is suitable for measurement of the In-Plane Radial Permeability (IPRP) of a porous material. The quantity of a saline solution (0.9% NaCl) flowing radially through an annular sample of the material under constant pressure is measured as a function of time.

Testing is performed at 23°C $\pm$ 2C° and a relative humidity 50% $\pm$ 5%. All samples are conditioned in this environment for twenty-four (24) hours before testing.

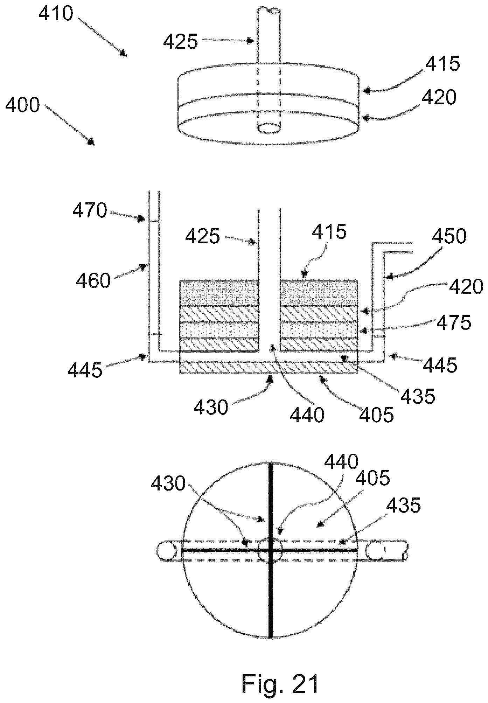

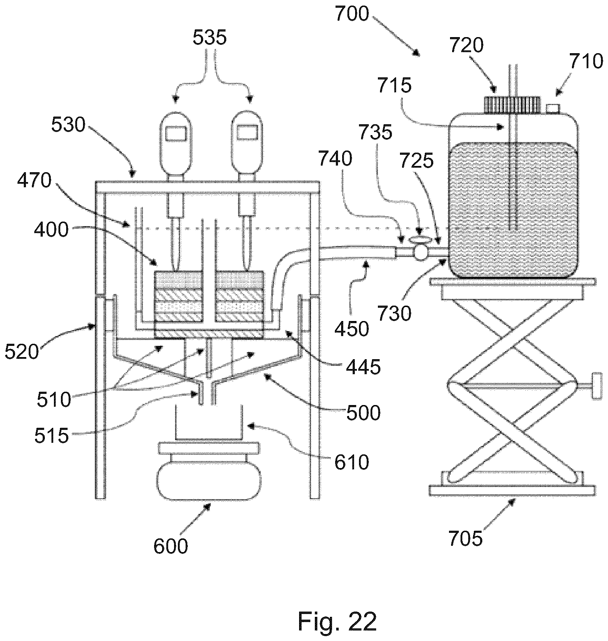

The apparatus -or parts thereof- as described below are shown in Figures 21 and 22.

The IPRP sample holder 400 is shown in FIG. 21 and comprises a cylindrical bottom plate 405, top plate 410, and cylindrical stainless-steel weight 415.

Top plate 410 comprises an annular base plate 420, which is 9 mm thick with an outer diameter of 70 mm and a tube 425 of 150 mm length fixed at the center thereof. The tube 425 has an outer diameter of 15.8 mm and an inner diameter of 12 mm. The tube is adhesively fixed into a circular 16 mm hole in the center of the base plate 420 such that the lower edge of the tube is flush with the lower surface of the base plate, as depicted in FIG. 21. The bottom plate 405 and top plate 410 are fabricated from Lexan® or equivalent. The stainless-steel weight 415 has an outer diameter of 70 mm and an inner diameter of 15.9 mm so that the weight is a close sliding fit on tube 425. The thickness of the stainless-steel weight 415 is approximately 22 mm and is adjusted so that the total weight of the top plate 410 and the stainless steel weight 415 is 687g $\pm$ 1g to provide 2.0 kPa of confining pressure during the measurement.

Bottom plate 405 is approximately 25 mm thick and has two registration grooves 430 cut into the lower surface of the plate such that each groove spans the diameter of the bottom plate and the grooves are perpendicular to each other. Each groove is 1.5 mm wide and 2 mm deep. Bottom plate 405 has a horizontal hole 435 which spans the diameter of the plate. The horizontal hole 435 has a diameter of 8 mm and its central axis is 15 mm below the upper surface of bottom plate 405. Bottom plate 405 also has a central vertical hole 440 which has a diameter of 8 mm and is 10 mm deep. The central hole 440 connects to the horizontal hole 435 to form a T-shaped cavity in the bottom plate 405. The outer portions of the horizontal hole 435 are threaded to accommodate pipe elbows 445 which are attached to the bottom plate 405 in a watertight fashion. One elbow is connected to a vertical transparent tube 460 with a total height of 175 mm measured from the bottom of bottom plate 405 (including elbow 445) and an internal diameter of 6 mm. The tube 460 is scribed with a suitable mark 470 at a height of 100 mm above the upper surface of the bottom plate 420. This is the reference for the fluid level to be maintained during the measurement. The other elbow 445 is connected to the fluid delivery reservoir 700 (described below) via a flexible tube.

A suitable fluid delivery reservoir 700 is shown in FIG. 22. Reservoir 700 is situated on a suitable laboratory jack 705 and has an air-tight stoppered opening 710 to facilitate filling of the reservoir with fluid. An open-ended glass tube 715 having an inner diameter of 10 mm extends through a port 720 in the top of the reservoir such that there is an airtight seal between the outside of the tube and the reservoir. Reservoir 700 is provided with an L-shaped delivery tube 725 having an inlet 730 that is below the surface of the fluid in the reservoir, a stopcock 735, and an outlet 740. The outlet 740 is connected to elbow 445 via flexible plastic tubing 450 (e.g., Tygon®). The internal diameter of the delivery tube 725, stopcock 735, and flexible plastic tubing 450 enable fluid delivery to the IPRP sample holder 400 at a high enough flow rate to maintain the level of fluid in tube 460 at the scribed mark 470 at all times during the measurement. The reservoir 700 has a capacity of approximately 6 liters, although larger reservoirs may be required depending on the sample thickness and permeability. Other fluid delivery systems may be employed provided that they are able to deliver the fluid to the sample holder 400 and maintain the level of fluid in tube 460 at the scribed mark 470 for the duration of the measurement.

The IPRP catchment funnel 500 is shown in FIG. 22 and comprises an outer housing 505 with an internal diameter at the upper edge of the funnel of approximately 125 mm. Funnel 500 is constructed such that liquid falling into the funnel drains rapidly and freely from spout 515. A stand with horizontal flange 520 around the funnel 500 facilitates mounting the funnel in a horizontal position. Two integral vertical internal ribs 510 span the internal diameter of the funnel and are perpendicular to each other. Each rib 510 is 1.5 mm wide and the top surfaces of the ribs lie in a horizontal plane. The funnel housing 500 and ribs 510 are fabricated from a suitably rigid material such as Lexan® or equivalent in order to support sample holder 400. To facilitate loading of the sample it is advantageous for the height of the ribs to be sufficient to allow the upper surface of the bottom plate 405 to lie above the funnel flange 520 when the bottom plate 405 is located on ribs 510. A bridge 530 is attached to flange 520 in order to mount two digital calipers 535 to measure the relative height of the stainless-steel weight 415. The digital calipers 535 have a resolution of $\pm$ 0.01 mm over a range of 25 mm. A suitable digital caliper is a Mitutoyo model 543-492B or equivalent. Each caliper is interfaced with a computer to allow height readings to be recorded periodically and stored electronically on the computer. Bridge 530 has a circular hole 22 mm in diameter to accommodate tube 425 without the tube touching the bridge.

Funnel 500 is mounted over an electronic balance 600, as shown in FIG. 22. The balance has a resolution of $\pm$ 0.01 g and a capacity of at least 1000 g. The balance 600 is also interfaced with a computer to allow the balance reading to be recorded periodically and stored electronically on the computer. A suitable balance is Mettler-Toledo model MS6002S or equivalent. A collection container 610 is situated on the balance pan so that liquid draining from the funnel spout 515 falls directly into the container 610.

The funnel 500 is mounted so that the upper surfaces of ribs 510 lie in a horizontal plane. Balance 600 and container 610 are positioned under the funnel 500 so that liquid draining from the funnel spout 515 falls directly into the container 610. The IPRP sample holder 400 is situated centrally in the funnel 500 with the ribs 510 located in grooves 430. The upper surface of the bottom plate 405 must be perfectly flat and level. The top plate 410 is aligned with and rests on the bottom plate 405. The stainless-steel weight 415 surrounds the tube 425 and rests on the top plate 410. Tube 425 extends vertically through the central hole in the bridge 530. Both calipers 535 are mounted firmly to the bridge 530 with the foot resting on a point on the upper surface of the stainless-steel weight 415. The calipers are set to zero in this state. The reservoir 700 is filled with 0.9% saline solution and re-sealed. The outlet 740 is connected to elbow 445 via flexible plastic tubing 450.

Sample Preparation

Remove the topsheet and any layer of the acquisition and distribution system from the complete absorbent article.

Retain the complete absorbent core. The backsheet can be retained to reduce the likelihood for disintegration of the absorbent core and ease handling of the sample (may not be required e.g., if the absorbent core is wrapped by a nonwoven web). However, the backsheet needs to be removed if the absorbent article has further components between the backsheet and the absorbent core. In such cases, all components need to be removed such as to isolate the absorbent core. Use die cutter to cut the sample at each location from the absorbent core in a donut shape having 70 mm - R0 exterior diameter and 12 mm - Ri interior diameter. Cut out samples from the crotch region 14 and the back region of the absorbent core.

The annular sample 475 of material to be tested has an outer diameter of 70 mm and an inner hole diameter of 12 mm. One suitable means of cutting the sample is to use a die cutter with sharp concentric blades.

The top plate 410 is lifted enough to insert the sample 475 between the top plate and the bottom plate 405 with the sample centered on the bottom plate and the plates aligned. The stopcock 735 is opened and the level of fluid in tube 460 is set to the scribed mark 470 by adjusting the height of the reservoir 700 using the jack 705 and by adjusting the position of the tube 715 in the reservoir.

Let the saline solution flow through the sample for pre-swelling for 30 minutes, making sure that the liquid column stays at the scribed mark 470.

After the 30 min pre-swelling and when the fluid level in the tube 460 is stable at the scribed mark 470 initiate recording data from the balance and calipers by the computer. Balance readings and time elapsed are recorded every 10 seconds for five minutes. The average sample thickness B is calculated from all caliper reading between 60 seconds and 300 seconds and expressed in cm. The flow rate in grams per second is the slope calculated by linear least squares regression fit of the balance reading (dependent variable) at different times (independent variable) considering only the readings between 60 seconds and 300 seconds.

Permeability k is then calculated by the following equation:

$$k = \frac{(Q/\rho_i) \cdot \mu \cdot \ln(R_0/R_i)}{2\pi \cdot B \cdot \Delta p} \tag{1}$$

Where:

k is the permeability ($cm^2$);
Q is the flow rate (g/s);
$\rho_1$ is the liquid density at 20°C ($g/cm^3$);
$\mu$ is the liquid viscosity at 20 °C (Pa·s);
$R_0$ is the outer sample radius (cm);
$R_i$ is the inner sample radius (cm);
B is the average sample thickness (cm); and
$\Delta p$ is the pressure drop (Pa) calculated according to the following equation:

$$\Delta p = \left(\Delta h - \frac{B}{2}\right) \cdot g \cdot \rho_l \tag{2}$$

Where:

$\Delta h$ is the height of the fluid level in the tube 460 from the top side of the bottom plate 405 to the mark 470; $\Delta h$ is 10cm +-0.1cm- (cm);
g is the acceleration constant ($m/s^2$); and
$\rho_1$ is the liquid density ($g/cm^3$).

**Opacity Test method**

Opacity by contrast ratio measurements are made using a 0°/45° spectrophotometer suitable for making standard CIE L*a*b* color measurements such as the Hunter ColorFlex EZ Spectrophotometer (Hunter Associates Laboratory Inc., Reston, Virginia, USA) or equivalent. The diameter of the instrument's measurement port is 30mm. Analyses are performed in a room controlled at about 23°C $\pm$ 2°C and 50%$\pm$2% relative humidity.

The instrument is calibrated per the vender instructions using standard black and white tiles provided by the instrument vendor. After calibration, the Y value of a standard white tile is measured and compared to its true value. The specified true Y value is of the standard white tile is typically in the range of 83 to 85, and the difference from true value should be 0.5 or less. The spectrophotometer is set to use the CIE XYZ color space, with a D65 standard illumination and 10° observer.

If possible, measurements are made on the lower ADS before it is integrated in an absorbent article. If this is not possible, care should be exerted when excising the lower ADS from the product not to impart any contamination or distortion to the test sample layer during the removal of the material from other layers (using cryogenic spray, such as Cyto-Freeze, Control Company, Houston, Texas, if needed). Five rectangular specimens of the lower ADS are taken such that each specimen center corresponds to the position of the loading point on the lower ADS and such that the length and width of each specimen are greater than the smallest dimension of the head.

The lower ADS specimen is positioned flat against the instrument with the outward facing surface toward the spectrophotometer's measurement port and the center of the lower ADS specimen (which corresponds to the loading point of the lower ADS) is matching with the center of the port. The specimen is further positioned such that no tears, holes or apertures are within the measurement port. The white standard tile is placed onto the opposing surface of the specimen such that it completely covers the portion of the specimen over the measurement port. A reading of XYZ values is taken, and each is recorded to the nearest 0.01. Without moving the specimen, the white plate is removed and replaced with the black standard plate. A second reading of XYZ values is taken and each is recorded to the nearest 0.01.

Opacity is calculated by dividing the Y value measured using the black tile as backing, divided by the Y value measured using the white tile as backing, then multiplying the ratio by 100.

$$Opacity\ [\%] = \frac{Y\ reading\ over\ black\ tile}{Y\ reading\ over\ white\ tile} \times 100\%$$

The five lower ADS specimens are analyzed in this way, and the Opacity of each is recorded. The arithmetic mean of the individual specimen results is calculated and reported as the Opacity in percentage to the nearest 1 %.

**Examples**

Comparative Example 1a: Pampers Harmonie diapers, Size 4, as commercially available in Germany in August 2024.

Comparative Example 1b: Pampers Harmonie diapers, Size 2, as commercially available in Germany in August 2024.

Comparative Example 2: Pampers Baby Dry diapers, Size 4, as commercially available in Germany in October 2022.

The commercial products Comparative Examples 1a, 1b and 2 had a topsheet, a backsheet, an absorbent core between the topsheet and the backsheet, and an acquisition and distribution system with two layers between the topsheet and the absorbent core. The first layer of the acquisition and distribution system was provided between the topsheet and the second layer of the acquisition and distribution system. The first layer of the acquisition and distribution system is a carded nonwoven web made of PET fibers and a liquid latex binder which had been cured after application onto the fibers to solidify. The second layer of the acquisition and distribution system was provided between the first layer of the acquisition and distribution system and the absorbent core. The second layer was a layer of intra-fiber crosslinked cellulose fibers.

Material Examples 1-3

Polyacrylic acid crosslinked cellulosic fibers with malic acid:
Polyacrylic acid crosslinked cellulosic fibers were made according to US11725339B2, except that a total chlorine free softwood feedstock was used and subjected to the crosslinking process according to US11725339B2.

Varying levels of aqueous malic acid (CAS 6915-15-7) were applied to the crosslinked cellulose fibers. A 50/50 wt% aqueous malic acid solution was prepared and pumped to a mixing nozzle where it further was diluted by the addition of water along with dilute aqueous hydrogen peroxide solution as bleaching agent, as described in US11725339B2. The mixed solution was sprayed onto the crosslinked fibers in cooling stages to achieve a desired amount of weight-% malic acid onto the fiber prior to compressing the malic acid treated crosslinked fibers into a bale.

Final malic acid amounts on the crosslinked cellulose fiber (in weight-% per total mass of the malic acid treated crosslinked cellulose fiber) were confirmed by extraction of malic acid from fiber with deionized water, followed by a sodium hydroxide (NaOH) titration.

Material Example 1 contained about 1.5 weight-% malic acid by weight of the total fiber.

Material Example 2 contained about 2.6 weight-% malic acid by weight of the total fiber.

Material Example 3 contained about 3.3 weight-% malic acid by weight of the total fiber.

Acquisition and Distribution System Examples 1-3

The Material Examples 1-3 were bale-opened, disintegrated, airlaid and glued onto an acquisition layer according to the process used in the production of Comparative Example 1a, Comparative Example 1b and Comparative Example 2.

The acquisition layer was a carded nonwoven web made of PET fibers and a liquid latex binder which had been cured after application onto the fibers to solidify with a basis weight of about 33 gsm.

Acquisition and Distribution System Example 1a contained Material Example 1, at basis weight of about 230 gsm.

Acquisition and Distribution System Example 1b contained Material Example 1, at basis weight of about 185 gsm.

Acquisition and Distribution System Example 2a contained Material Example 2, at basis weight of about 230 gsm.

Acquisition and Distribution System Example 2b contained Material Example 2, at basis weight of about 185 gsm.

Acquisition and Distribution System Example 3a contained Material Example 3 at basis weight of about 230 gsm.

Acquisition and Distribution System Example 3b contained Material Example 3, at basis weight of about 185 gsm.

Absorbent Article Examples 1a-3a

Same as Comparative Example 1a, but first the topsheet and then the acquisition and distribution system was carefully removed (though it was not needed here, it is generally possible to use ice spray to separate the layers to ease the separation, e.g., non-flammable quick coolant IT ICER Green (PRF)).

For Absorbent Article Examples 1a to 3a, the removed acquisition and distribution system was replaced with the Acquisition and Distribution System Examples 1a to 3a, respectively.

Absorbent Article Examples 1a contained Acquisition and Distribution System Example 1a.

Absorbent Article Examples 2a contained Acquisition and Distribution System Example 2a.

Absorbent Article Examples 3a contained Acquisition and Distribution System Example 3a.

Afterwards the article was reassembled by reapplying the topsheet.

Absorbent Article Examples 1b-3b

Same as Comparative Example 1b, but first the topsheet and then the acquisition and distribution system was carefully removed (though it was not needed here, it is generally possible to use ice spray to separate the layers to ease the separation, e.g., non-flammable quick coolant IT ICER Green (PRF).

For Absorbent Article Examples 1b to 3b, the removed acquisition and distribution system was replaced with the Acquisition and Distribution System Examples 1b to 3b, respectively.

Absorbent Article Examples 1b contained Acquisition and Distribution System Example 1b.

Absorbent Article Examples 2b contained Acquisition and Distribution System Example 2b.

Absorbent Article Examples 3b contained Acquisition and Distribution System Example 3b.

Afterwards the article was reassembled by reapplying the topsheet.

The second layer of the acquisition and distribution system of the commercial samples and Acquisition and Distribution System Examples 1a,b to 3a,b were subjected to the Acid Equivalence method.

Table 1: Results for Examples 1 a,b to 3 a,b and Comparative Examples 1a,b and 2

|  | Acid Equivalence value [mmol/g] | $pH_0$ | Conductivity [mS/cm] |
|---|---|---|---|
| Example 1a | 0.219 | 3.9 | 0.11 |
| Example 1b | 0.219 | 3.9 | 0.11 |
| Example 2a | 0.388 | 3.7 | 0.16 |
| Example 2b | 0.388 | 3.7 | 0.16 |
| Example 3a | 0.456 | 3.6 | 0.19 |
| Example 3b | 0.456 | 3.6 | 0.19 |
| Comparative Example 1a | 0.014 | 6.1 | 0.03 |
| Comparative Example 1b | 0.014 | 6.1 | 0.03 |
| Comparative Example 2 | 0.018 | 6.2 | 0.03 |

The Absorbent Article Examples 1a,b to 3a,b and the Comparative Examples 1a and 1b were subjected to the Urease Activity method, with the following modification of the test method:

3 replicates were measured instead of 6 replicates.
For Comparative Example 1b (size 2 diaper), 40 ml instead of 75 ml test liquid was applied.

Table 2: Results of topsheet pH of the Absorbent Article Examples 1a,b to 3a,b and the Comparative Examples 1a and 1b

|  | topsheet pH of the modified Urease Activity method |
|---|---|
| Absorbent Article Example 1a | 6.3 |
| Absorbent Article Example 2a | 6.3 |
| Absorbent Article Example 3a | 6.0 |
| Absorbent Article Example 1b | 6.2 |
| Absorbent Article Example 2b | 5.6 |
| Absorbent Article Example 3b | 5.7 |
| Comparative Example 1a | 7.0 |
| Comparative Example 1b | 6.9 |

Comparative Examples 1a and 1b show relatively high topsheet pH values compared to Examples 1a, 2a, 3a, 1b, 2b and 3b. The topsheet pH for Examples 1a, 2a, 3a, 1b, 2b and 3b is acidic and within or close to the skin pH range.

Contemplated Examples

A1. Absorbent article (10) comprising

a liquid permeable topsheet (26),
a liquid impermeable backsheet (28),
an absorbent core (30), and
an acquisition and distribution system (38) comprising an acquisition and/ or distribution layer;
wherein the absorbent core (30) is provided between the topsheet (26) and the backsheet (28); the acquisition and distribution system (38) is provided between the topsheet (26) and the absorbent core (30);
wherein the acquisition and/or distribution layer exhibits an Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein.

A2. The absorbent article (10) of example A1, wherein the acquisition and/or distribution layer comprises one or more monomeric acids.

A3. The absorbent article (10) of example A2, wherein the acquisition and/or distribution layer comprises one or more monomeric organic acids.

A4. The absorbent article (10) of example A3, wherein the acquisition and/or distribution layer comprises one or more monomeric acids selected from the group consisting of carboxylic acids, amino acids and sulfonic acids.

A5. The absorbent article (10) of example A4, wherein the acquisition and/or distribution layer comprises one or more monomeric carboxylic acids.

A6. The absorbent article (10) of any one of examples A1 to A5, wherein the acquisition and/or distribution layer comprises one or more diprotonic and/or triprotonic acids.

A7. The absorbent article (10) of any one of examples A3 to A6, wherein the acquisition and/or distribution layer comprises one or more non-ethylenical acids.

A8. The absorbent article (10) of any one of example A2 to A7, wherein the one or more monomeric acids and/ or diprotonic and/or triprotonic acids are distributed throughout the at least one acquisition and/ or distribution layer.

A9. The absorbent article (10) of example A8, wherein the one or more monomeric acids and/ or diprotonic and/or triprotonic acids are distributed throughout the at least one acquisition and/ or distribution layer by applying the one or more acids to at least part of the fibers forming the at least one acquisition and/ or distribution layer prior to or during forming the at least one acquisition and/ or distribution layer.

A10. The absorbent article (10) of any one of examples A1 to A9, wherein the acquisition and distribution system (38) comprises natural fibers, and/or comprises cellulose-based fibers.

A11. The absorbent article (10) of any one of examples A1 to A10, wherein the acquisition and distribution system (38) comprises a nonwoven layer, preferably a carded nonwoven layer.

A12. The absorbent article (10) of any one of examples A1 to A11, wherein the acquisition and distribution system (38) comprises a first acquisition and/ or distribution layer and a second acquisition and/ or distribution layer, wherein the second acquisition and/ or distribution layer is provided between the first acquisition and/ or distribution layer and the absorbent core (30) and the first acquisition and/ or distribution layer is provided between the topsheet and the second acquisition and/ or distribution layer; wherein the first acquisition and/ or distribution layer comprises a nonwoven material and/ or the second acquisition and/ or distribution layer comprises a cross-linked cellulosic material.

A13. The absorbent article (10) of example A12, wherein the one or more monomeric acids and/ or diprotonic and/or triprotonic acids are distributed throughout the second acquisition and/ or distribution layer.

A14. The absorbent article (10) of example A13, wherein one or more monomeric acids and/ or diprotonic and/or triprotonic acids are distributed throughout the second acquisition and/ or distribution layer by applying the one or more monomeric acids and/ or diprotonic and/or triprotonic acids after and/ or during the step of crosslinking the cellulosic material.

A15. The absorbent article (10) of any one of examples A12 to A14, wherein the second acquisition and/ or distribution layer comprises the one or more monomeric acids and/ or diprotonic and/or triprotonic acids, while the first acquisition and/ or distribution layer is substantially free of the one or more monomeric acids and/ or diprotonic and/or triprotonic acids.

A16. The absorbent article (10) of any one of examples A1 to A15, wherein the acquisition and/ or distribution layer comprises malic acid.

A17. The absorbent article (10) of any one of examples A1 to A16, wherein the acquisition and/ or distribution layer and/ or the acquisition and distribution system (38) comprises substantially no free acids except malic acid, acrylic acid, oligoacrylic acid, polyacrylic acid or combinations thereof or salts thereof.

A18. The absorbent article (10) of any one of examples A1 to A17, wherein the comprised acquisition and/or distribution layer and/ or the acquisition and/or distribution material have a $pH_0$ value of about 5.5 or lower, or of about 5.0 or lower, or of about 4.5 or lower, or of about 4.0 or lower according to the $pH_0$ Test Method disclosed herein.

A19. The absorbent article (10) of any one of examples A1 to A18, wherein the comprised acquisition and/or distribution layer and/ or the acquisition and/or distribution material have a $pH_0$ value of about 2.5 or higher, or of about 3.0 or higher, or between 3.0 and 5.5, or between 3.5 and 5.0 according to the $pH_0$ Test Method disclosed herein.

A20. The absorbent article (10) of any one of examples A1 to A19, wherein the comprised acquisition and/or distribution layer and/ or the acquisition and/or distribution material have a conductivity value of about 0.05 mS/cm or higher, or of about 0.1 mS/cm or higher, or between 0.1 mS/cm and 0.2 mS/cm according to the Conductivity Test Method disclosed herein.

A21. A process for making an absorbent article (10) comprising

a liquid permeable topsheet (26),
a liquid impermeable backsheet (28),
an absorbent core (30), and
an acquisition and distribution system (38) comprising an acquisition and/ or distribution layer;
wherein the absorbent core (30) is provided between the topsheet (26) and the backsheet (28); the acquisition and distribution system (38) is provided between the topsheet (26) and the absorbent core (30);

wherein the acquisition and distribution system (38) comprises one or more acquisition and/or distribution layers; wherein the process comprises the step of:

a) applying one or more monomeric acids to the one or more acquisition and/or distribution layers of the acquisition and distribution system (38) in an amount such that the acquisition and distribution system (38) exhibits an Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein;

wherein the one or more monomeric acids are applied to the one or more acquisition and/or distribution layers of the acquisition and distribution system (38) prior to the acquisition and distribution system (38) being provided between the topsheet (26) and the absorbent core (30).

A22. The process for making an absorbent article (10) of example A21, wherein the one or more monomeric acids each are organic acids.

A23. The process for making an absorbent article (10) of example A22, wherein the one or more organic acids each are selected from the group consisting of carboxylic acids, amino acids and/or sulfonic acids.

A24. The process for making an absorbent article (10) of example A23, wherein the one or more organic acids each are carboxylic acids.

A25. The process for making an absorbent article (10) of any one of examples A21 to A25, wherein the one or more monomeric acids each are diprotonic and/or triprotonic acids.

A26. The process for making an absorbent article (10) of example any one of examples A21 to A26, wherein the one or more carboxylic acids each are selected from the group consisting of malic acid, succinic acid or lactic acid.

A27. The process for making an absorbent article (10) of any one of any one of examples A21 to A26, wherein the one or more monomeric acids each are applied to the acquisition and distribution system (38) in a solution and/ or suspension.

A28. The process for making an absorbent article (10) of any one of any one of examples A21 to A27, wherein the one or more monomeric acids are applied to at least part of the fibers forming at least one acquisition and/ or distribution layer of the acquisition and distribution system (38) prior to the at least one acquisition and/ or distribution layer being formed.

A29. The process for making an absorbent article (10) of any one of any one of examples A21 to A28, wherein at least one acquisition and/ or distribution layer comprises cross-linked cellulosic material.

A30. The process for making an absorbent article (10) of example A29, wherein the one or more monomeric acids are applied to cellulosic fibers after and/ or during the step of crosslinking the cellulosic material.

A31. The process for making an absorbent article (10) of any one of examples A21 to A30, wherein the acquisition and distribution system (38) and/ or the comprised acquisition and/or distribution layers are exposed to a heating and/or drying step for not more than 7 hours during the process, or not subjected to any heating and/or drying step.

A32. The absorbent article (10) of any one of Examples A1-A20, wherein the acquisition and distribution system (38) comprises an acquisition and/or distribution layer having a body-facing surface and an opposing garment-facing surface; the layer has a surface pH value of the body-facing surface (pH_bfs) and a surface pH value of the garment-facing surface (pH_gfs), wherein
the pH_bfs and the pH_gfs differ by at most 0.2, preferably by at most 0.1, or more preferably by at most 0.05 according to the Surface pH Test Method as set out herein.

A33. The absorbent article (10) of any one of one of Examples A1-A20 or A32, wherein the acquisition and distribution system (38) comprises one or more acquisition and/or distribution layers with a basis weight of about 40 gsm or less.

A. Absorbent article (10) comprising

a liquid permeable topsheet (26),

a liquid impermeable backsheet (28),

an absorbent core (30), and

an acquisition and distribution system (38) comprising an acquisition and/ or distribution layer;

wherein the absorbent core (30) is provided between the topsheet (26) and the backsheet (28); the acquisition and distribution system (38) is provided between the topsheet (26) and the absorbent core (30);

wherein an acquisition and/ or distribution layer comprises an acquisition and/or distribution material that exhibits an Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein.

B. The absorbent article (10) of example A, wherein the acquisition and/or distribution material comprises one or more monomeric acids.

C. The absorbent article (10) of example B, wherein the acquisition and/or distribution material comprises one or more monomeric organic acids.

D. The absorbent article (10) of example C, wherein the acquisition and/or distribution material comprises one or more monomeric organic acids selected from the group consisting of carboxylic acids, amino acids and sulfonic acids.

E. The absorbent article (10) of example A to D, wherein the acquisition and/or distribution material comprises one or more carboxylic acids.

F. The absorbent article (10) of any one of examples A to E, wherein the acquisition and/or distribution material comprises one or more diprotonic and/or triprotonic acids.

G. The absorbent article (10) of any one of examples C to F, wherein the acquisition and/or distribution material comprises one or more non-ethylenical acids.

H. The absorbent article (10) of any one of examples A to G, wherein the acquisition and/or distribution material is distributed throughout the acquisition and/ or distribution layer.

I. The absorbent article (10) of examples G or H, wherein the one or more monomeric non-ethylenical acids is comprised by the acquisition and/or distribution material prior to forming the acquisition and/ or distribution layer.

J. The absorbent article (10) of any one of examples A-D, wherein the acquisition and/or distribution material comprises natural fibers, and/or comprises cellulose-based fibers.

K. The absorbent article (10) of any one of examples A-J, wherein the acquisition and/ or distribution layer comprises at least 10%, preferably at least 20%, more preferably at least 30%, even more preferably at least 40%, more preferably at least 50%, more preferably at least 60%, more preferably at least 70%, more preferably at least 80% or even at least 90% of the acquisition and/or distribution material by weight of the acquisition and/ or distribution layer or even consists of the acquisition and/or distribution material.

L. The absorbent article (10) of any one of examples A-K, wherein the acquisition and distribution system (38) comprises a nonwoven layer.

M. The absorbent article (10) of examples L, wherein the acquisition and distribution system (38) comprises a carded nonwoven layer.

N. The absorbent article (10) of any one of examples A-M, wherein the acquisition and distribution system (38) comprises a first acquisition and/ or distribution layer and a second acquisition and/ or distribution layer, wherein the second acquisition and/ or distribution layer is provided between the first acquisition and/ or distribution layer and the absorbent core (30) and the first acquisition and/ or distribution layer is provided between the topsheet and the second acquisition and/ or distribution layer.

O. The absorbent article (10) of example N, wherein the first acquisition and/ or distribution layer comprises a nonwoven material and/ or the second acquisition and/ or distribution layer comprises a cross-linked cellulosic material.

P. The absorbent article (10) of examples N or O, wherein the acquisition and distribution material is distributed throughout the second acquisition and/ or distribution layer.

Q. The absorbent article (10) of any one of examples I or J, wherein the acquisition and distribution material comprises cross-linked cellulosic material and one or more non-ethylenical monomeric acids.

R. The absorbent article (10) of example Q, wherein the one or more non-ethylenical acids are applied to the cross-linked cellulosic material after and/ or during the step of crosslinking the cellulosic material.

S. The absorbent article (10) of any one of examples J-R, wherein the second acquisition and/ or distribution layer comprises the acquisition and/or distribution material, while the first acquisition and/ or distribution layer is substantially free of the acquisition and distribution material.

T. The absorbent article (10) of any one of Examples A-S, wherein the acquisition and/or distribution material comprises malic acid, succinic acid and/ or lactic acid, preferably malic acid.

U. The absorbent article (10) of any one of the Examples A-T, wherein the acquisition and/or distribution material comprises substantially no free acids except malic acid, acrylic acid, oligoacrylic acid, polyacrylic acid or combinations thereof or salts thereof.

V. The absorbent article (10) of any one of Examples A-U, wherein the comprised acquisition and/or distribution layers and/ or the acquisition and/or distribution material have a $pH_0$ value of about 5.5 or lower, or of about 5.0 or lower, or of about 4.5 or lower, or of about 4.0 or lower according to the $pH_0$ Test Method disclosed herein.

W. The absorbent article (10) of any one of Examples A-V, wherein the comprised acquisition and/or distribution layers and/ or the acquisition and/or distribution material have a $pH_0$ value of about 2.5 or higher, or of about 3.0 or higher, or between 3.0 and 5.5, or between 3.5 and 5.0 according to the $pH_0$ Test Method disclosed herein.

X. The absorbent article (10) of any one any one of Examples A-W, wherein the comprised acquisition and/or distribution layers and/ or the acquisition and/or distribution material have a conductivity value of about 0.05 mS/cm or higher, or of about 0.1 mS/cm or higher, or between 0.1 mS/cm and 0.2 mS/cm according to the Conductivity Test Method disclosed herein.

Y. A process for making an absorbent article (10) comprising

> a liquid permeable topsheet (26),
> a liquid impermeable backsheet (28),
> an absorbent core (30), and
> an acquisition and distribution system (38) comprising an acquisition and/ or distribution layer;
> wherein the absorbent core (30) is provided between the topsheet (26) and the backsheet (28); the acquisition and distribution system (38) is provided between the topsheet (26) and the absorbent core (30);
> wherein the process comprises the step of:
>
>> a) providing an acquisition and/ or distribution layer comprising an acquisition and/ or distribution material wherein the acquisition and/ or distribution material exhibits Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein.

Z. The process for making an absorbent article (10) of Example Y, wherein one or more monomeric acids are applied to the acquisition and/or distribution material prior to the acquisition and distribution system (38) being provided between the topsheet (26) and the absorbent core (30).

AA. The process for making an absorbent article (10) of Example Z, wherein the one or more monomeric acids comprise malic acid, succinic acid and/ or lactic acid.

BB. The process for making an absorbent article (10) of any one of Examples Y-AA, wherein the acquisition and/ or distribution material comprises cross-linked cellulosic material.

CC. The process for making an absorbent article (10) of Example AA, wherein the one or more monomeric acids are applied to cellulosic fibers after and/ or during the step of crosslinking the cellulosic material.

DD. The process for making an absorbent article (10) of any one of Examples Y-CC, wherein the acquisition and distribution system (38) and/ or the comprised acquisition and/or distribution material are exposed to a heating and/or drying step for not more than 7 hours during the process, or not subjected to any heating and/or drying step.

EE. The absorbent article (10) of any one of Examples A-X, wherein the acquisition and distribution system (38) comprises an acquisition and/or distribution layer having a body-facing surface and an opposing garment-facing surface; the layer has a surface pH value of the body-facing surface (pH_bfs) and a surface pH value of the garment-facing surface (pH_gfs), wherein
the pH_bfs and the pH_gfs differ by at most 0.2, preferably by at most 0.1, or more preferably by at most 0.05 according to the Surface pH Test Method as set out herein.

FF. The absorbent article (10) of any one of one of Examples A-X or EE, wherein the acquisition and distribution system (38) comprises one or more acquisition and/or distribution layers with a basis weight of about 40 gsm or less.

GG. The process for making an absorbent article (10) of any one of Examples Y-DD, wherein the acquisition and distribution system (38) comprises one or more acquisition and/or distribution layers with a basis weight of about 40 gsm or less.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. Absorbent article (10) comprising

    a liquid permeable topsheet (26),
    a liquid impermeable backsheet (28),
    an absorbent core (30), and
    an acquisition and distribution system (38) comprising an acquisition and/ or distribution layer;
    wherein the absorbent core (30) is provided between the topsheet (26) and the backsheet (28); the acquisition and distribution system (38) is provided between the topsheet (26) and the absorbent core (30);
    wherein the acquisition and/or distribution layer exhibits an Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein.

2. The absorbent article (10) of claim 1, wherein the acquisition and/or distribution layer comprises one or more monomeric acids, preferably one or more monomeric organic acids.

3. The absorbent article (10) of claim 2, wherein the acquisition and/or distribution layer comprises one or more monomeric acids selected from the group consisting of carboxylic acids, amino acids and sulfonic acids, preferably one or more monomeric carboxylic acids.

4. The absorbent article (10) of claims 2 or 3, wherein the one or more monomeric acids are distributed throughout the at least one acquisition and/ or distribution layer by applying the one or more acids to at least part of the fibers forming the at least one acquisition and/ or distribution layer prior to or during forming the at least one acquisition and/ or distribution layer.

5. The absorbent article (10) of any one of the preceding claims wherein the acquisition and distribution system (38) comprises natural fibers, and/or comprises cellulose-based fibers.

6. The absorbent article (10) of any one of the preceding claims wherein the acquisition and distribution system (38) comprises a nonwoven layer, preferably a carded nonwoven layer.

7. The absorbent article (10) of any one of the preceding claims, wherein the acquisition and distribution system (38) comprises a first acquisition and/ or distribution layer and a second acquisition and/ or distribution layer, wherein the second acquisition and/ or distribution layer is provided between the first acquisition and/ or distribution layer and the absorbent core (30) and the first acquisition and/ or distribution layer is provided between the topsheet and the second acquisition and/ or distribution layer;
wherein the first acquisition and/ or distribution layer comprises a nonwoven material and/ or the second acquisition and/ or distribution layer comprises a cross-linked cellulosic material.

8. The absorbent article (10) of claim 7, wherein the one or more monomeric acids are distributed throughout the second acquisition and/ or distribution layer by applying the one or more monomeric acids after and/ or during the step of crosslinking the cellulosic material.

9. The absorbent article (10) of claims 7 or 8, wherein the second acquisition and/ or distribution layer comprises the one or more monomeric acids, while the first acquisition and/ or distribution layer is substantially free of the one or more monomeric acids.

10. The absorbent article (10) of any one of the preceding claims, wherein the acquisition and/ or distribution layer comprises malic acid.

11. The absorbent article (10) of any one of the preceding claims, wherein the acquisition and/ or distribution layer and/ or the acquisition and distribution system (38) comprises substantially no free acids except malic acid, acrylic acid, oligoacrylic acid, polyacrylic acid or combinations thereof or salts thereof.

12. The absorbent article (10) of any one of the preceding claims, wherein the acquisition and distribution system (38) comprises one or more acquisition and/or distribution layer with a basis weight of about 40 gsm or less.

13. A process for making an absorbent article (10) comprising

> a liquid permeable topsheet (26),
> a liquid impermeable backsheet (28),
> an absorbent core (30), and
> an acquisition and distribution system (38) comprising an acquisition and/ or distribution layer;
> wherein the absorbent core (30) is provided between the topsheet (26) and the backsheet (28); the acquisition and distribution system (38) is provided between the topsheet (26) and the absorbent core (30);
> wherein the acquisition and distribution system (38) comprises one or more acquisition and/or distribution layers;
> wherein the process comprises the step of:
> b) applying one or more monomeric acids to the acquisition and/or distribution layer in an amount such that acquisition and/or distribution layer exhibits an Acid Equivalent value of from 0.050 mmol/g to 6.000 mmol/g, or from 0.100 mmol/g to 3.000 mmol/g, or from 0.150 mmol/g to 2.000 mmol/g, or from 0.180 mmol/g to 1.000 mmol/g, or from 0.200 mmol/g to 0.850 mmol/g, or from 0.220 mmol/g to 0.500 mmol/g according to the Acid Equivalent method disclosed herein;

wherein the one or more monomeric acids are applied to the one or more acquisition and/or distribution layers of the acquisition and distribution system (38) prior to the acquisition and distribution system (38) being provided between the topsheet (26) and the absorbent core (30).

14. The process for making an absorbent article (10) of claim 13, wherein the one or more monomeric acids each are organic acids, preferably selected from the group consisting of carboxylic acids, amino acids and/or sulfonic acids, more preferably are carboxylic acids.

15. The process for making an absorbent article (10) of any one of claims 13 or 14, wherein the one or more monomeric acids are applied to at least part of the fibers forming at least one acquisition and/ or distribution layer of the acquisition and distribution system (38) prior to the at least one acquisition and/ or distribution layer being formed.

16. The process for making an absorbent article (10) of any one of claims 13-15, wherein the at least one acquisition and/ or distribution layer comprises cross-linked cellulosic material and the one or more monomeric acids are applied to cellulosic fibers after and/ or during the step of crosslinking the cellulosic material.

17. The process for making an absorbent article (10) of any one of claims 13-16, wherein the acquisition and distribution system (38) and/ or the comprised acquisition and/or distribution layers are exposed to a heating and/or drying step for not more than 7 hours during the process, or not subjected to any heating and/or drying step.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

EP 4 721 713 A1

Fig. 16

Fig. 17A

Fig. 17B

Fig. 17C

Fig. 17D

Fig. 17E

Fig. 18A

Fig. 18B

EP 4 721 713 A1

1850

1800

1830

1810

1820

Fig. 19

Fig. 20

Fig. 21

Fig. 22

Europäisches
Patentamt
European
Patent Office
Office européen
des brevets

# EUROPEAN SEARCH REPORT

**Application Number**

EP 24 20 3929

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 4 442 232 A1 (PROCTER & GAMBLE [US]) 9 October 2024 (2024-10-09) * paragraphs [0229] - [0230]; example 16 * * paragraphs [0127] - [0131] * * paragraph [0228] * * examples 1-15 * * page 34 * | 1-7, 10-14 | INV. A61F13/15 A61F13/537 A61F13/84 A61L15/26 A61L15/46 |
| X A | WO 00/35502 A1 (SCA HYGIENE PROD AB [SE]; FORSGREN BRUSK ULLA [SE]; RUNEMAN BO [SE]) 22 June 2000 (2000-06-22) * claim 1; figure 1 * * page 5, lines 23-26 * * page 6, lines 12-18, 25-31 * * page 7, lines 3-4 * * page 8, line 17 - page 9, line 2 * | 1-5, 10-17 7-9 | |
| A | US 2012/220968 A1 (CONFALONE PHILIP [US] ET AL) 30 August 2012 (2012-08-30) * the whole document * | 1-17 | |
| A | US 2023/046382 A1 (BÄHRLE CHRISTIAN [DE] ET AL) 16 February 2023 (2023-02-16) * the whole document * | 1-17 | TECHNICAL FIELDS SEARCHED (IPC) A61F A61L |
| A | US 2019/254889 A1 (STRIDFELDT CHATRINE [SE] ET AL) 22 August 2019 (2019-08-22) * the whole document * | 1-17 | |

The present search report has been drawn up for all claims

| Place of search | Date of completion of the search | Examiner |
|---|---|---|
| Munich | 14 February 2025 | Beckert, Audrey |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 20 3929

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2025

| Patent document cited in search report | | Publication date | Patent family member(s) | | Publication date |
|---|---|---|---|---|---|
| EP 4442232 | A1 | 09-10-2024 | EP | 4442232 A1 | 09-10-2024 |
| | | | US | 2024335336 A1 | 10-10-2024 |
| | | | WO | 2024211340 A1 | 10-10-2024 |
| WO 0035502 | A1 | 22-06-2000 | AT | E318621 T1 | 15-03-2006 |
| | | | AU | 761090 B2 | 29-05-2003 |
| | | | BR | 9916291 A | 16-10-2001 |
| | | | CA | 2352308 A1 | 22-06-2000 |
| | | | CO | 5241358 A1 | 31-01-2003 |
| | | | DE | 69930162 T2 | 23-11-2006 |
| | | | EP | 1140226 A1 | 10-10-2001 |
| | | | ES | 2259244 T3 | 16-09-2006 |
| | | | JP | 4671508 B2 | 20-04-2011 |
| | | | JP | 2002532153 A | 02-10-2002 |
| | | | KR | 20010101183 A | 14-11-2001 |
| | | | PL | 348917 A1 | 17-06-2002 |
| | | | RU | 2230576 C2 | 20-06-2004 |
| | | | SK | 8012001 A3 | 03-12-2001 |
| | | | TN | SN99241 A1 | 31-12-2001 |
| | | | US | 6649806 B1 | 18-11-2003 |
| | | | WO | 0035502 A1 | 22-06-2000 |
| | | | ZA | 200104232 B | 23-08-2002 |
| US 2012220968 | A1 | 30-08-2012 | CA | 2766646 A1 | 28-08-2012 |
| | | | CN | 102650094 A | 29-08-2012 |
| | | | EP | 2491910 A1 | 29-08-2012 |
| | | | JP | 2012179355 A | 20-09-2012 |
| | | | RU | 2012106807 A | 27-08-2013 |
| | | | US | 2012220968 A1 | 30-08-2012 |
| US 2023046382 | A1 | 16-02-2023 | AU | 2020406737 A1 | 30-06-2022 |
| | | | BR | 112022012176 A2 | 06-09-2022 |
| | | | CN | 114828798 A | 29-07-2022 |
| | | | EP | 3838243 A1 | 23-06-2021 |
| | | | EP | 4076322 A1 | 26-10-2022 |
| | | | ES | 2968610 T3 | 13-05-2024 |
| | | | JP | 2023520955 A | 23-05-2023 |
| | | | PL | 4076322 T3 | 06-05-2024 |
| | | | US | 2023046382 A1 | 16-02-2023 |
| | | | WO | 2021123085 A1 | 24-06-2021 |
| US 2019254889 | A1 | 22-08-2019 | AU | 2017329216 A1 | 07-03-2019 |
| | | | BR | 112019005659 A2 | 04-06-2019 |
| | | | CN | 109475446 A | 15-03-2019 |
| | | | CO | 2019003443 A2 | 12-04-2019 |
| | | | EP | 3515387 A1 | 31-07-2019 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 24 20 3929

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2025

| Patent document cited in search report | Publication date | Patent family member(s) | | Publication date |
|---|---|---|---|---|
| | | JP | 6871363 B2 | 12-05-2021 |
| | | JP | 2019528919 A | 17-10-2019 |
| | | RU | 2711302 C1 | 16-01-2020 |
| | | US | 2019254889 A1 | 22-08-2019 |
| | | WO | 2018054766 A1 | 29-03-2018 |

For more details about this annex : see Official Journal of the European Patent Office, No. 12/82

page 2 of 2

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9011402 B, Roe
- US 20130211363
- US 7901393 B
- US 9498389 A
- US 6645190 B
- US 8747379 B
- US 8372052 B
- US 8361048 B
- US 6761711 B
- US 6817994 B
- US 8007485 B
- US 7862550 B
- US 6969377 B
- US 7497851 B
- US 6849067 B
- US 6893426 B
- US 6953452 B

- US 6840928 B
- US 8579876 B
- US 7682349 B
- US 7156833 B
- US 7201744 B
- US 20070219521 A1
- US 20070219521
- US 8703450 B
- US 9630901 B
- US 9822197 B
- US 20110139657
- US 20110139658 A
- US 20110152812 A
- US 20160206774 A
- US 9169366 B
- US 11725339 B2

**Non-patent literature cited in the description**

- **DAWSON, R.M.C. et al.** Biochemical Research. Clarendon Press, 1959

- **BRAUDE, E.A.** ; **F.C. NACHOD**. Determination of Organic Structures by Physical Methods. Academic Press, 1955
- *CHEMICAL ABSTRACTS*, 6915-15-7